# EUROPEAN PATENT APPLICATION

(11) **EP 3 907 289 A1**
(43) Date of publication of application: **10.11.2021**
(21) Application number: 19893472.1
(22) Date of filing: 29.11.2019
(51) Int. Cl.: C12N 15/55, C12N 1/15, C12N 1/19, C12N 1/21, C12N 5/10, C12N 9/16, C12P 7/40

(54) **MODIFIED CHRYSANTHEMIC ACID ESTERASE**

(30) Priority: 06.12.2018 JP 2018229479
(71) Applicant: Amano Enzyme Inc., Nagoya-shi Aichi 460-8630 (JP)
(72) Inventor: ONO Atsushi, Kakamigahara-shi, Gifu 509-0109 (JP); YOSHIDA Kazunori, Kakamigahara-shi, Gifu 509-0109 (JP)
(74) Representative: J A Kemp LLP
(86) International application number: PCT/JP2019/046690
(87) International publication number: WO 2020/116331

(57) **Abstract**

The present invention aims to provide an esterase useful for the production of (1R,3S)-chrysanthemic acid. A plurality of amino acid substitutions effective for improving the reactivity with (1R,3S)-ethyl chrysanthemate are disclosed in an esterase derived from a microorganism of the genus Arthrobacter.

SEQUENCE LISTING

<110>  AMANO ENZYME INC.

<120>  MODIFIED ESTERASE

<130>  AE17007P

<150>  JP P2018-229479
<151>  2018-12-06

<160>  23

<170>  PatentIn version 3.5

<210>  1
<211>  375
<212>  PRT
<213>  Arthrobacter globiformis

<400>  1

Val Asp Ala Gln Thr Ile Ala Pro Gly Phe Glu Ser Val Ala Glu Leu
1               5                   10                  15

Phe Gly Arg Phe Leu Ser Glu Asp Arg Glu Tyr Ser Ala Gln Leu Ala
            20                  25                  30

Ala Tyr His Arg Gly Val Lys Val Leu Asp Ile Ser Gly Gly Pro His
        35                  40                  45

Arg Arg Pro Asp Ser Val Thr Gly Val Phe Ser Cys Ser Lys Gly Val
    50                  55                  60

Ser Gly Leu Val Ile Ala Leu Leu Val Gln Asp Gly Phe Leu Asp Leu
65                  70                  75                  80

Asp Ala Glu Val Val Lys Tyr Trp Pro Glu Phe Gly Ala Glu Gly Ser
                85                  90                  95

Ala Thr Ile Thr Val Ala Gln Leu Leu Ser His Gln Ala Gly Leu Leu
            100                 105                 110

Gly Val Glu Gly Gly Leu Thr Leu Ala Glu Tyr Asn Asn Ser Glu Leu
        115                 120                 125

Ala Ala Ala Lys Leu Ala Gln Met Arg Pro Leu Trp Lys Pro Gly Thr
    130                 135                 140

Ala Phe Gly Tyr His Ala Leu Thr Ile Gly Val Phe Met Glu Glu Leu
145                 150                 155                 160

Cys Arg Arg Ile Thr Gly Ser Thr Leu Gln Glu Ile Tyr Glu Gln Arg

                165                 170                 175

Ile Arg Ser Val Thr Gly Ala His Phe Phe Leu Gly Leu Pro Glu Ser
            180                 185                 190

Glu Glu Pro Arg Tyr Ala Thr Leu Arg Trp Ala Ala Asp Pro Ser Gln
        195                 200                 205

Pro Trp Ile Asp Pro Ala Ser His Phe Gly Leu Ser Ala Asn Ser Ala
    210                 215                 220

Val Gly Asp Ile Leu Asp Leu Pro Asn Leu Arg Glu Val Arg Ala Ala
225                 230                 235                 240

Gly Leu Ser Ser Ala Ala Gly Val Ala Ser Ala Glu Gly Met Ala Arg
                245                 250                 255

Val Tyr Ala Ala Ala Leu Thr Gly Leu Ala Ala Asn Gly Asp Arg Ala
            260                 265                 270

Ala Val Ala Pro Leu Leu Ser Glu Glu Thr Ile Gln Thr Val Thr Ala
        275                 280                 285

Glu Gln Val Phe Gly Ile Asp Arg Val Phe Gly Glu Thr Ser Cys Phe
    290                 295                 300

Gly Thr Val Phe Met Lys Ser His Ala Arg Ser Pro Tyr Gly Ser Tyr
305                 310                 315                 320

Arg Ala Phe Gly His Asp Gly Ala Ser Ala Ser Leu Gly Phe Ala Asp
                325                 330                 335

Pro Val Tyr Glu Leu Ala Phe Gly Tyr Val Pro Gln Gln Ala Glu Pro
            340                 345                 350

Gly Gly Ala Gly Cys Arg Asn Leu Glu Leu Ser Ala Ala Val Arg Lys
        355                 360                 365

Ala Val Thr Glu Leu Ala Gln
    370                 375

<210> 2
<211> 375
<212> PRT
<213> Artificial Sequence

<220>
<223> Mutant A328G

<400>  2

Val Asp Ala Gln Thr Ile Ala Pro Gly Phe Glu Ser Val Ala Glu Leu
1               5                   10                  15

Phe Gly Arg Phe Leu Ser Glu Asp Arg Glu Tyr Ser Ala Gln Leu Ala
            20                  25                  30

Ala Tyr His Arg Gly Val Lys Val Leu Asp Ile Ser Gly Gly Pro His
        35                  40                  45

Arg Arg Pro Asp Ser Val Thr Gly Val Phe Ser Cys Ser Lys Gly Val
    50                  55                  60

Ser Gly Leu Val Ile Ala Leu Leu Val Gln Asp Gly Phe Leu Asp Leu
65                  70                  75                  80

Asp Ala Glu Val Val Lys Tyr Trp Pro Glu Phe Gly Ala Glu Gly Ser
                85                  90                  95

Ala Thr Ile Thr Val Ala Gln Leu Leu Ser His Gln Ala Gly Leu Leu
            100                 105                 110

Gly Val Glu Gly Gly Leu Thr Leu Ala Glu Tyr Asn Asn Ser Glu Leu
        115                 120                 125

Ala Ala Ala Lys Leu Ala Gln Met Arg Pro Leu Trp Lys Pro Gly Thr
    130                 135                 140

Ala Phe Gly Tyr His Ala Leu Thr Ile Gly Val Phe Met Glu Glu Leu
145                 150                 155                 160

Cys Arg Arg Ile Thr Gly Ser Thr Leu Gln Glu Ile Tyr Glu Gln Arg
                165                 170                 175

Ile Arg Ser Val Thr Gly Ala His Phe Phe Leu Gly Leu Pro Glu Ser
            180                 185                 190

Glu Glu Pro Arg Tyr Ala Thr Leu Arg Trp Ala Ala Asp Pro Ser Gln
        195                 200                 205

Pro Trp Ile Asp Pro Ala Ser His Phe Gly Leu Ser Ala Asn Ser Ala
    210                 215                 220

Val Gly Asp Ile Leu Asp Leu Pro Asn Leu Arg Glu Val Arg Ala Ala
225                 230                 235                 240

Gly Leu Ser Ser Ala Ala Gly Val Ala Ser Ala Glu Gly Met Ala Arg

245                 250                 255

Val Tyr Ala Ala Ala Leu Thr Gly Leu Ala Ala Asn Gly Asp Arg Ala
            260                 265                 270

Ala Val Ala Pro Leu Leu Ser Glu Glu Thr Ile Gln Thr Val Thr Ala
        275                 280                 285

Glu Gln Val Phe Gly Ile Asp Arg Val Phe Gly Glu Thr Ser Cys Phe
    290                 295                 300

Gly Thr Val Phe Met Lys Ser His Ala Arg Ser Pro Tyr Gly Ser Tyr
305                 310                 315                 320

Arg Ala Phe Gly His Asp Gly Gly Ser Ala Ser Leu Gly Phe Ala Asp
                325                 330                 335

Pro Val Tyr Glu Leu Ala Phe Gly Tyr Val Pro Gln Gln Ala Glu Pro
            340                 345                 350

Gly Gly Ala Gly Cys Arg Asn Leu Glu Leu Ser Ala Ala Val Arg Lys
        355                 360                 365

Ala Val Thr Glu Leu Ala Gln
    370                 375

<210>  3
<211>  375
<212>  PRT
<213>  Artificial Sequence

<220>
<223>  Mutant A328G/F217W

<400>  3

Val Asp Ala Gln Thr Ile Ala Pro Gly Phe Glu Ser Val Ala Glu Leu
1               5                   10                  15

Phe Gly Arg Phe Leu Ser Glu Asp Arg Glu Tyr Ser Ala Gln Leu Ala
            20                  25                  30

Ala Tyr His Arg Gly Val Lys Val Leu Asp Ile Ser Gly Gly Pro His
        35                  40                  45

Arg Arg Pro Asp Ser Val Thr Gly Val Phe Ser Cys Ser Lys Gly Val
    50                  55                  60

Ser Gly Leu Val Ile Ala Leu Leu Val Gln Asp Gly Phe Leu Asp Leu
65                  70                  75                  80

Asp Ala Glu Val Val Lys Tyr Trp Pro Glu Phe Gly Ala Glu Gly Ser
                85                  90                  95

Ala Thr Ile Thr Val Ala Gln Leu Leu Ser His Gln Ala Gly Leu Leu
            100                 105                 110

Gly Val Glu Gly Gly Leu Thr Leu Ala Glu Tyr Asn Asn Ser Glu Leu
        115                 120                 125

Ala Ala Ala Lys Leu Ala Gln Met Arg Pro Leu Trp Lys Pro Gly Thr
    130                 135                 140

Ala Phe Gly Tyr His Ala Leu Thr Ile Gly Val Phe Met Glu Glu Leu
145                 150                 155                 160

Cys Arg Arg Ile Thr Gly Ser Thr Leu Gln Glu Ile Tyr Glu Gln Arg
                165                 170                 175

Ile Arg Ser Val Thr Gly Ala His Phe Phe Leu Gly Leu Pro Glu Ser
            180                 185                 190

Glu Glu Pro Arg Tyr Ala Thr Leu Arg Trp Ala Ala Asp Pro Ser Gln
        195                 200                 205

Pro Trp Ile Asp Pro Ala Ser His Trp Gly Leu Ser Ala Asn Ser Ala
    210                 215                 220

Val Gly Asp Ile Leu Asp Leu Pro Asn Leu Arg Glu Val Arg Ala Ala
225                 230                 235                 240

Gly Leu Ser Ser Ala Ala Gly Val Ala Ser Ala Glu Gly Met Ala Arg
                245                 250                 255

Val Tyr Ala Ala Ala Leu Thr Gly Leu Ala Ala Asn Gly Asp Arg Ala
            260                 265                 270

Ala Val Ala Pro Leu Leu Ser Glu Glu Thr Ile Gln Thr Val Thr Ala
        275                 280                 285

Glu Gln Val Phe Gly Ile Asp Arg Val Phe Gly Glu Thr Ser Cys Phe
    290                 295                 300

Gly Thr Val Phe Met Lys Ser His Ala Arg Ser Pro Tyr Gly Ser Tyr
305                 310                 315                 320

Arg Ala Phe Gly His Asp Gly Gly Ser Ala Ser Leu Gly Phe Ala Asp

                325                 330                 335

Pro Val Tyr Glu Leu Ala Phe Gly Tyr Val Pro Gln Gln Ala Glu Pro
            340                 345                 350

Gly Gly Ala Gly Cys Arg Asn Leu Glu Leu Ser Ala Ala Val Arg Lys
        355                 360                 365

Ala Val Thr Glu Leu Ala Gln
    370                 375

<210> 4
<211> 375
<212> PRT
<213> Artificial Sequence

<220>
<223> Mutant A328G/A221F

<400> 4

Val Asp Ala Gln Thr Ile Ala Pro Gly Phe Glu Ser Val Ala Glu Leu
1               5                   10                  15

Phe Gly Arg Phe Leu Ser Glu Asp Arg Glu Tyr Ser Ala Gln Leu Ala
            20                  25                  30

Ala Tyr His Arg Gly Val Lys Val Leu Asp Ile Ser Gly Gly Pro His
        35                  40                  45

Arg Arg Pro Asp Ser Val Thr Gly Val Phe Ser Cys Ser Lys Gly Val
    50                  55                  60

Ser Gly Leu Val Ile Ala Leu Leu Val Gln Asp Gly Phe Leu Asp Leu
65                  70                  75                  80

Asp Ala Glu Val Val Lys Tyr Trp Pro Glu Phe Gly Ala Glu Gly Ser
                85                  90                  95

Ala Thr Ile Thr Val Ala Gln Leu Leu Ser His Gln Ala Gly Leu Leu
            100                 105                 110

Gly Val Glu Gly Gly Leu Thr Leu Ala Glu Tyr Asn Asn Ser Glu Leu
        115                 120                 125

Ala Ala Ala Lys Leu Ala Gln Met Arg Pro Leu Trp Lys Pro Gly Thr
    130                 135                 140

Ala Phe Gly Tyr His Ala Leu Thr Ile Gly Val Phe Met Glu Glu Leu
145                 150                 155                 160

Cys Arg Arg Ile Thr Gly Ser Thr Leu Gln Glu Ile Tyr Glu Gln Arg
                165                 170                 175

Ile Arg Ser Val Thr Gly Ala His Phe Phe Leu Gly Leu Pro Glu Ser
            180                 185                 190

Glu Glu Pro Arg Tyr Ala Thr Leu Arg Trp Ala Ala Asp Pro Ser Gln
        195                 200                 205

Pro Trp Ile Asp Pro Ala Ser His Phe Gly Leu Ser Phe Asn Ser Ala
    210                 215                 220

Val Gly Asp Ile Leu Asp Leu Pro Asn Leu Arg Glu Val Arg Ala Ala
225                 230                 235                 240

Gly Leu Ser Ser Ala Ala Gly Val Ala Ser Ala Glu Gly Met Ala Arg
                245                 250                 255

Val Tyr Ala Ala Ala Leu Thr Gly Leu Ala Ala Asn Gly Asp Arg Ala
            260                 265                 270

Ala Val Ala Pro Leu Leu Ser Glu Glu Thr Ile Gln Thr Val Thr Ala
        275                 280                 285

Glu Gln Val Phe Gly Ile Asp Arg Val Phe Gly Glu Thr Ser Cys Phe
    290                 295                 300

Gly Thr Val Phe Met Lys Ser His Ala Arg Ser Pro Tyr Gly Ser Tyr
305                 310                 315                 320

Arg Ala Phe Gly His Asp Gly Gly Ser Ala Ser Leu Gly Phe Ala Asp
                325                 330                 335

Pro Val Tyr Glu Leu Ala Phe Gly Tyr Val Pro Gln Gln Ala Glu Pro
            340                 345                 350

Gly Gly Ala Gly Cys Arg Asn Leu Glu Leu Ser Ala Ala Val Arg Lys
        355                 360                 365

Ala Val Thr Glu Leu Ala Gln
    370                 375

<210>  5
<211>  375
<212>  PRT
<213>  Artificial Sequence

<220>
<223>  Mutant A328G/A221M

<400>  5

Val Asp Ala Gln Thr Ile Ala Pro Gly Phe Glu Ser Val Ala Glu Leu
1               5                   10                  15

Phe Gly Arg Phe Leu Ser Glu Asp Arg Glu Tyr Ser Ala Gln Leu Ala
            20                  25                  30

Ala Tyr His Arg Gly Val Lys Val Leu Asp Ile Ser Gly Gly Pro His
        35                  40                  45

Arg Arg Pro Asp Ser Val Thr Gly Val Phe Ser Cys Ser Lys Gly Val
    50                  55                  60

Ser Gly Leu Val Ile Ala Leu Leu Val Gln Asp Gly Phe Leu Asp Leu
65                  70                  75                  80

Asp Ala Glu Val Val Lys Tyr Trp Pro Glu Phe Gly Ala Glu Gly Ser
                85                  90                  95

Ala Thr Ile Thr Val Ala Gln Leu Leu Ser His Gln Ala Gly Leu Leu
            100                 105                 110

Gly Val Glu Gly Gly Leu Thr Leu Ala Glu Tyr Asn Asn Ser Glu Leu
        115                 120                 125

Ala Ala Ala Lys Leu Ala Gln Met Arg Pro Leu Trp Lys Pro Gly Thr
    130                 135                 140

Ala Phe Gly Tyr His Ala Leu Thr Ile Gly Val Phe Met Glu Glu Leu
145                 150                 155                 160

Cys Arg Arg Ile Thr Gly Ser Thr Leu Gln Glu Ile Tyr Glu Gln Arg
                165                 170                 175

Ile Arg Ser Val Thr Gly Ala His Phe Phe Leu Gly Leu Pro Glu Ser
            180                 185                 190

Glu Glu Pro Arg Tyr Ala Thr Leu Arg Trp Ala Ala Asp Pro Ser Gln
        195                 200                 205

Pro Trp Ile Asp Pro Ala Ser His Phe Gly Leu Ser Met Asn Ser Ala
    210                 215                 220

Val Gly Asp Ile Leu Asp Leu Pro Asn Leu Arg Glu Val Arg Ala Ala
225                 230                 235                 240

Gly Leu Ser Ser Ala Ala Gly Val Ala Ser Ala Glu Gly Met Ala Arg
                245                 250                 255

Val Tyr Ala Ala Ala Leu Thr Gly Leu Ala Ala Asn Gly Asp Arg Ala
            260                 265                 270

Ala Val Ala Pro Leu Leu Ser Glu Glu Thr Ile Gln Thr Val Thr Ala
        275                 280                 285

Glu Gln Val Phe Gly Ile Asp Arg Val Phe Gly Glu Thr Ser Cys Phe
    290                 295                 300

Gly Thr Val Phe Met Lys Ser His Ala Arg Ser Pro Tyr Gly Ser Tyr
305                 310                 315                 320

Arg Ala Phe Gly His Asp Gly Gly Ser Ala Ser Leu Gly Phe Ala Asp
                325                 330                 335

Pro Val Tyr Glu Leu Ala Phe Gly Tyr Val Pro Gln Gln Ala Glu Pro
            340                 345                 350

Gly Gly Ala Gly Cys Arg Asn Leu Glu Leu Ser Ala Ala Val Arg Lys
        355                 360                 365

Ala Val Thr Glu Leu Ala Gln
    370                 375

<210>  6
<211>  375
<212>  PRT
<213>  Artificial Sequence

<220>
<223>  Mutant A328G/A221L

<400>  6

Val Asp Ala Gln Thr Ile Ala Pro Gly Phe Glu Ser Val Ala Glu Leu
1               5                   10                  15

Phe Gly Arg Phe Leu Ser Glu Asp Arg Glu Tyr Ser Ala Gln Leu Ala
            20                  25                  30

Ala Tyr His Arg Gly Val Lys Val Leu Asp Ile Ser Gly Gly Pro His
        35                  40                  45

Arg Arg Pro Asp Ser Val Thr Gly Val Phe Ser Cys Ser Lys Gly Val
    50                  55                  60

Ser Gly Leu Val Ile Ala Leu Leu Val Gln Asp Gly Phe Leu Asp Leu
65                  70                  75                  80

Asp Ala Glu Val Val Lys Tyr Trp Pro Glu Phe Gly Ala Glu Gly Ser
                85                  90                  95

Ala Thr Ile Thr Val Ala Gln Leu Leu Ser His Gln Ala Gly Leu Leu
            100                 105                 110

Gly Val Glu Gly Gly Leu Thr Leu Ala Glu Tyr Asn Asn Ser Glu Leu
        115                 120                 125

Ala Ala Ala Lys Leu Ala Gln Met Arg Pro Leu Trp Lys Pro Gly Thr
    130                 135                 140

Ala Phe Gly Tyr His Ala Leu Thr Ile Gly Val Phe Met Glu Glu Leu
145                 150                 155                 160

Cys Arg Arg Ile Thr Gly Ser Thr Leu Gln Glu Ile Tyr Glu Gln Arg
                165                 170                 175

Ile Arg Ser Val Thr Gly Ala His Phe Phe Leu Gly Leu Pro Glu Ser
            180                 185                 190

Glu Glu Pro Arg Tyr Ala Thr Leu Arg Trp Ala Ala Asp Pro Ser Gln
        195                 200                 205

Pro Trp Ile Asp Pro Ala Ser His Phe Gly Leu Ser Leu Asn Ser Ala
    210                 215                 220

Val Gly Asp Ile Leu Asp Leu Pro Asn Leu Arg Glu Val Arg Ala Ala
225                 230                 235                 240

Gly Leu Ser Ser Ala Ala Gly Val Ala Ser Ala Glu Gly Met Ala Arg
                245                 250                 255

Val Tyr Ala Ala Ala Leu Thr Gly Leu Ala Ala Asn Gly Asp Arg Ala
            260                 265                 270

Ala Val Ala Pro Leu Leu Ser Glu Glu Thr Ile Gln Thr Val Thr Ala
        275                 280                 285

Glu Gln Val Phe Gly Ile Asp Arg Val Phe Gly Glu Thr Ser Cys Phe
    290                 295                 300

Gly Thr Val Phe Met Lys Ser His Ala Arg Ser Pro Tyr Gly Ser Tyr
305                 310                 315                 320

Arg Ala Phe Gly His Asp Gly Gly Ser Ala Ser Leu Gly Phe Ala Asp
                325                 330                 335

Pro Val Tyr Glu Leu Ala Phe Gly Tyr Val Pro Gln Gln Ala Glu Pro
            340                 345                 350

Gly Gly Ala Gly Cys Arg Asn Leu Glu Leu Ser Ala Ala Val Arg Lys
        355                 360                 365

Ala Val Thr Glu Leu Ala Gln
    370                 375

<210>  7
<211>  375
<212>  PRT
<213>  Artificial Sequence

<220>
<223>  Mutant A328G/A221V

<400>  7

Val Asp Ala Gln Thr Ile Ala Pro Gly Phe Glu Ser Val Ala Glu Leu
1               5                   10                  15

Phe Gly Arg Phe Leu Ser Glu Asp Arg Glu Tyr Ser Ala Gln Leu Ala
            20                  25                  30

Ala Tyr His Arg Gly Val Lys Val Leu Asp Ile Ser Gly Gly Pro His
        35                  40                  45

Arg Arg Pro Asp Ser Val Thr Gly Val Phe Ser Cys Ser Lys Gly Val
    50                  55                  60

Ser Gly Leu Val Ile Ala Leu Leu Val Gln Asp Gly Phe Leu Asp Leu
65                  70                  75                  80

Asp Ala Glu Val Val Lys Tyr Trp Pro Glu Phe Gly Ala Glu Gly Ser
                85                  90                  95

Ala Thr Ile Thr Val Ala Gln Leu Leu Ser His Gln Ala Gly Leu Leu
            100                 105                 110

Gly Val Glu Gly Gly Leu Thr Leu Ala Glu Tyr Asn Asn Ser Glu Leu
        115                 120                 125

Ala Ala Ala Lys Leu Ala Gln Met Arg Pro Leu Trp Lys Pro Gly Thr
    130                 135                 140

Ala Phe Gly Tyr His Ala Leu Thr Ile Gly Val Phe Met Glu Glu Leu
145                 150                 155                 160

Cys Arg Arg Ile Thr Gly Ser Thr Leu Gln Glu Ile Tyr Glu Gln Arg
                165                 170                 175

Ile Arg Ser Val Thr Gly Ala His Phe Phe Leu Gly Leu Pro Glu Ser
            180                 185                 190

Glu Glu Pro Arg Tyr Ala Thr Leu Arg Trp Ala Ala Asp Pro Ser Gln
        195                 200                 205

Pro Trp Ile Asp Pro Ala Ser His Phe Gly Leu Ser Val Asn Ser Ala
    210                 215                 220

Val Gly Asp Ile Leu Asp Leu Pro Asn Leu Arg Glu Val Arg Ala Ala
225                 230                 235                 240

Gly Leu Ser Ser Ala Ala Gly Val Ala Ser Ala Glu Gly Met Ala Arg
                245                 250                 255

Val Tyr Ala Ala Ala Leu Thr Gly Leu Ala Ala Asn Gly Asp Arg Ala
            260                 265                 270

Ala Val Ala Pro Leu Leu Ser Glu Glu Thr Ile Gln Thr Val Thr Ala
        275                 280                 285

Glu Gln Val Phe Gly Ile Asp Arg Val Phe Gly Glu Thr Ser Cys Phe
    290                 295                 300

Gly Thr Val Phe Met Lys Ser His Ala Arg Ser Pro Tyr Gly Ser Tyr
305                 310                 315                 320

Arg Ala Phe Gly His Asp Gly Gly Ser Ala Ser Leu Gly Phe Ala Asp
                325                 330                 335

Pro Val Tyr Glu Leu Ala Phe Gly Tyr Val Pro Gln Gln Ala Glu Pro
            340                 345                 350

Gly Gly Ala Gly Cys Arg Asn Leu Glu Leu Ser Ala Ala Val Arg Lys
        355                 360                 365

Ala Val Thr Glu Leu Ala Gln
    370                 375

<210>  8
<211>  375

<212>  PRT
<213>  Artificial Sequence

<220>
<223>  Mutant A328G/I228L

<400>  8

Val Asp Ala Gln Thr Ile Ala Pro Gly Phe Glu Ser Val Ala Glu Leu
1               5                   10                  15

Phe Gly Arg Phe Leu Ser Glu Asp Arg Glu Tyr Ser Ala Gln Leu Ala
            20                  25                  30

Ala Tyr His Arg Gly Val Lys Val Leu Asp Ile Ser Gly Gly Pro His
        35                  40                  45

Arg Arg Pro Asp Ser Val Thr Gly Val Phe Ser Cys Ser Lys Gly Val
    50                  55                  60

Ser Gly Leu Val Ile Ala Leu Leu Val Gln Asp Gly Phe Leu Asp Leu
65                  70                  75                  80

Asp Ala Glu Val Val Lys Tyr Trp Pro Glu Phe Gly Ala Glu Gly Ser
                85                  90                  95

Ala Thr Ile Thr Val Ala Gln Leu Leu Ser His Gln Ala Gly Leu Leu
            100                 105                 110

Gly Val Glu Gly Gly Leu Thr Leu Ala Glu Tyr Asn Asn Ser Glu Leu
        115                 120                 125

Ala Ala Ala Lys Leu Ala Gln Met Arg Pro Leu Trp Lys Pro Gly Thr
    130                 135                 140

Ala Phe Gly Tyr His Ala Leu Thr Ile Gly Val Phe Met Glu Glu Leu
145                 150                 155                 160

Cys Arg Arg Ile Thr Gly Ser Thr Leu Gln Glu Ile Tyr Glu Gln Arg
                165                 170                 175

Ile Arg Ser Val Thr Gly Ala His Phe Phe Leu Gly Leu Pro Glu Ser
            180                 185                 190

Glu Glu Pro Arg Tyr Ala Thr Leu Arg Trp Ala Ala Asp Pro Ser Gln
        195                 200                 205

Pro Trp Ile Asp Pro Ala Ser His Phe Gly Leu Ser Ala Asn Ser Ala
    210                 215                 220

Val Gly Asp Leu Leu Asp Leu Pro Asn Leu Arg Glu Val Arg Ala Ala
225                 230                 235                 240

Gly Leu Ser Ser Ala Ala Gly Val Ala Ser Ala Glu Gly Met Ala Arg
                245                 250                 255

Val Tyr Ala Ala Ala Leu Thr Gly Leu Ala Ala Asn Gly Asp Arg Ala
            260                 265                 270

Ala Val Ala Pro Leu Leu Ser Glu Glu Thr Ile Gln Thr Val Thr Ala
        275                 280                 285

Glu Gln Val Phe Gly Ile Asp Arg Val Phe Gly Glu Thr Ser Cys Phe
    290                 295                 300

Gly Thr Val Phe Met Lys Ser His Ala Arg Ser Pro Tyr Gly Ser Tyr
305                 310                 315                 320

Arg Ala Phe Gly His Asp Gly Gly Ser Ala Ser Leu Gly Phe Ala Asp
                325                 330                 335

Pro Val Tyr Glu Leu Ala Phe Gly Tyr Val Pro Gln Gln Ala Glu Pro
            340                 345                 350

Gly Gly Ala Gly Cys Arg Asn Leu Glu Leu Ser Ala Ala Val Arg Lys
        355                 360                 365

Ala Val Thr Glu Leu Ala Gln
    370                 375

<210>  9
<211>  375
<212>  PRT
<213>  Artificial Sequence

<220>
<223>  Mutant A328G/A221F/D326V

<400>  9

Val Asp Ala Gln Thr Ile Ala Pro Gly Phe Glu Ser Val Ala Glu Leu
1               5                   10                  15

Phe Gly Arg Phe Leu Ser Glu Asp Arg Glu Tyr Ser Ala Gln Leu Ala
            20                  25                  30

Ala Tyr His Arg Gly Val Lys Val Leu Asp Ile Ser Gly Gly Pro His
        35                  40                  45

Arg Arg Pro Asp Ser Val Thr Gly Val Phe Ser Cys Ser Lys Gly Val
    50                  55                  60

Ser Gly Leu Val Ile Ala Leu Leu Val Gln Asp Gly Phe Leu Asp Leu
65                  70                  75                  80

Asp Ala Glu Val Val Lys Tyr Trp Pro Glu Phe Gly Ala Glu Gly Ser
                85                  90                  95

Ala Thr Ile Thr Val Ala Gln Leu Leu Ser His Gln Ala Gly Leu Leu
            100                 105                 110

Gly Val Glu Gly Gly Leu Thr Leu Ala Glu Tyr Asn Asn Ser Glu Leu
        115                 120                 125

Ala Ala Ala Lys Leu Ala Gln Met Arg Pro Leu Trp Lys Pro Gly Thr
    130                 135                 140

Ala Phe Gly Tyr His Ala Leu Thr Ile Gly Val Phe Met Glu Glu Leu
145                 150                 155                 160

Cys Arg Arg Ile Thr Gly Ser Thr Leu Gln Glu Ile Tyr Glu Gln Arg
                165                 170                 175

Ile Arg Ser Val Thr Gly Ala His Phe Phe Leu Gly Leu Pro Glu Ser
            180                 185                 190

Glu Glu Pro Arg Tyr Ala Thr Leu Arg Trp Ala Ala Asp Pro Ser Gln
        195                 200                 205

Pro Trp Ile Asp Pro Ala Ser His Phe Gly Leu Ser Phe Asn Ser Ala
    210                 215                 220

Val Gly Asp Ile Leu Asp Leu Pro Asn Leu Arg Glu Val Arg Ala Ala
225                 230                 235                 240

Gly Leu Ser Ser Ala Ala Gly Val Ala Ser Ala Glu Gly Met Ala Arg
                245                 250                 255

Val Tyr Ala Ala Ala Leu Thr Gly Leu Ala Ala Asn Gly Asp Arg Ala
            260                 265                 270

Ala Val Ala Pro Leu Leu Ser Glu Glu Thr Ile Gln Thr Val Thr Ala
        275                 280                 285

Glu Gln Val Phe Gly Ile Asp Arg Val Phe Gly Glu Thr Ser Cys Phe
    290                 295                 300

Gly Thr Val Phe Met Lys Ser His Ala Arg Ser Pro Tyr Gly Ser Tyr
305                 310                 315                 320

Arg Ala Phe Gly His Val Gly Gly Ser Ala Ser Leu Gly Phe Ala Asp
                325                 330                 335

Pro Val Tyr Glu Leu Ala Phe Gly Tyr Val Pro Gln Gln Ala Glu Pro
            340                 345                 350

Gly Gly Ala Gly Cys Arg Asn Leu Glu Leu Ser Ala Ala Val Arg Lys
        355                 360                 365

Ala Val Thr Glu Leu Ala Gln
    370                 375

<210>  10
<211>  375
<212>  PRT
<213>  Artificial Sequence

<220>
<223>  Mutant A328G/A221F/D326V/F298L

<400>  10

Val Asp Ala Gln Thr Ile Ala Pro Gly Phe Glu Ser Val Ala Glu Leu
1               5                   10                  15

Phe Gly Arg Phe Leu Ser Glu Asp Arg Glu Tyr Ser Ala Gln Leu Ala
            20                  25                  30

Ala Tyr His Arg Gly Val Lys Val Leu Asp Ile Ser Gly Gly Pro His
        35                  40                  45

Arg Arg Pro Asp Ser Val Thr Gly Val Phe Ser Cys Ser Lys Gly Val
    50                  55                  60

Ser Gly Leu Val Ile Ala Leu Leu Val Gln Asp Gly Phe Leu Asp Leu
65                  70                  75                  80

Asp Ala Glu Val Val Lys Tyr Trp Pro Glu Phe Gly Ala Glu Gly Ser
                85                  90                  95

Ala Thr Ile Thr Val Ala Gln Leu Leu Ser His Gln Ala Gly Leu Leu
            100                 105                 110

Gly Val Glu Gly Gly Leu Thr Leu Ala Glu Tyr Asn Asn Ser Glu Leu
        115                 120                 125

Ala Ala Ala Lys Leu Ala Gln Met Arg Pro Leu Trp Lys Pro Gly Thr
    130                 135                 140

Ala Phe Gly Tyr His Ala Leu Thr Ile Gly Val Phe Met Glu Glu Leu
145                 150                 155                 160

Cys Arg Arg Ile Thr Gly Ser Thr Leu Gln Glu Ile Tyr Glu Gln Arg
                165                 170                 175

Ile Arg Ser Val Thr Gly Ala His Phe Phe Leu Gly Leu Pro Glu Ser
            180                 185                 190

Glu Glu Pro Arg Tyr Ala Thr Leu Arg Trp Ala Ala Asp Pro Ser Gln
        195                 200                 205

Pro Trp Ile Asp Pro Ala Ser His Phe Gly Leu Ser Phe Asn Ser Ala
    210                 215                 220

Val Gly Asp Ile Leu Asp Leu Pro Asn Leu Arg Glu Val Arg Ala Ala
225                 230                 235                 240

Gly Leu Ser Ser Ala Ala Gly Val Ala Ser Ala Glu Gly Met Ala Arg
                245                 250                 255

Val Tyr Ala Ala Ala Leu Thr Gly Leu Ala Ala Asn Gly Asp Arg Ala
            260                 265                 270

Ala Val Ala Pro Leu Leu Ser Glu Glu Thr Ile Gln Thr Val Thr Ala
        275                 280                 285

Glu Gln Val Phe Gly Ile Asp Arg Val Leu Gly Glu Thr Ser Cys Phe
    290                 295                 300

Gly Thr Val Phe Met Lys Ser His Ala Arg Ser Pro Tyr Gly Ser Tyr
305                 310                 315                 320

Arg Ala Phe Gly His Val Gly Gly Ser Ala Ser Leu Gly Phe Ala Asp
                325                 330                 335

Pro Val Tyr Glu Leu Ala Phe Gly Tyr Val Pro Gln Gln Ala Glu Pro
            340                 345                 350

Gly Gly Ala Gly Cys Arg Asn Leu Glu Leu Ser Ala Ala Val Arg Lys
        355                 360                 365

Ala Val Thr Glu Leu Ala Gln
    370                 375

<210>  11
<211>  375
<212>  PRT
<213>  Artificial Sequence

<220>
<223>  Mutant A328G/A221F/D326V/F298L/N222D

<400>  11

Val Asp Ala Gln Thr Ile Ala Pro Gly Phe Glu Ser Val Ala Glu Leu
1               5                   10                  15

Phe Gly Arg Phe Leu Ser Glu Asp Arg Glu Tyr Ser Ala Gln Leu Ala
            20                  25                  30

Ala Tyr His Arg Gly Val Lys Val Leu Asp Ile Ser Gly Gly Pro His
        35                  40                  45

Arg Arg Pro Asp Ser Val Thr Gly Val Phe Ser Cys Ser Lys Gly Val
    50                  55                  60

Ser Gly Leu Val Ile Ala Leu Leu Val Gln Asp Gly Phe Leu Asp Leu
65                  70                  75                  80

Asp Ala Glu Val Val Lys Tyr Trp Pro Glu Phe Gly Ala Glu Gly Ser
                85                  90                  95

Ala Thr Ile Thr Val Ala Gln Leu Leu Ser His Gln Ala Gly Leu Leu
            100                 105                 110

Gly Val Glu Gly Gly Leu Thr Leu Ala Glu Tyr Asn Asn Ser Glu Leu
        115                 120                 125

Ala Ala Ala Lys Leu Ala Gln Met Arg Pro Leu Trp Lys Pro Gly Thr
    130                 135                 140

Ala Phe Gly Tyr His Ala Leu Thr Ile Gly Val Phe Met Glu Glu Leu
145                 150                 155                 160

Cys Arg Arg Ile Thr Gly Ser Thr Leu Gln Glu Ile Tyr Glu Gln Arg
                165                 170                 175

Ile Arg Ser Val Thr Gly Ala His Phe Phe Leu Gly Leu Pro Glu Ser
            180                 185                 190

Glu Glu Pro Arg Tyr Ala Thr Leu Arg Trp Ala Ala Asp Pro Ser Gln
        195                 200                 205

Pro Trp Ile Asp Pro Ala Ser His Phe Gly Leu Ser Phe Asp Ser Ala
    210                 215                 220

Val Gly Asp Ile Leu Asp Leu Pro Asn Leu Arg Glu Val Arg Ala Ala
225                 230                 235                 240

Gly Leu Ser Ser Ala Ala Gly Val Ala Ser Ala Glu Gly Met Ala Arg
                245                 250                 255

Val Tyr Ala Ala Ala Leu Thr Gly Leu Ala Ala Asn Gly Asp Arg Ala
            260                 265                 270

Ala Val Ala Pro Leu Leu Ser Glu Glu Thr Ile Gln Thr Val Thr Ala
        275                 280                 285

Glu Gln Val Phe Gly Ile Asp Arg Val Leu Gly Glu Thr Ser Cys Phe
    290                 295                 300

Gly Thr Val Phe Met Lys Ser His Ala Arg Ser Pro Tyr Gly Ser Tyr
305                 310                 315                 320

Arg Ala Phe Gly His Val Gly Gly Ser Ala Ser Leu Gly Phe Ala Asp
                325                 330                 335

Pro Val Tyr Glu Leu Ala Phe Gly Tyr Val Pro Gln Gln Ala Glu Pro
            340                 345                 350

Gly Gly Ala Gly Cys Arg Asn Leu Glu Leu Ser Ala Ala Val Arg Lys
        355                 360                 365

Ala Val Thr Glu Leu Ala Gln
    370                 375

<210>  12
<211>  1128
<212>  DNA
<213>  Arthrobacter globiformis

<400>  12
gtggatgcac agacgattgc ccctggattc gaatcagtcg ccgaactctt tggccgtttc        60

ctgagcgaag accgggaata ttcagcccag ctcgcggcct accaccgcgg agtcaaggta       120

ttggacatca gcggtgggcc gcaccgccgc ccggattccg tgaccggtgt tttctcctgc       180

tccaagggag tatccgggct ggtcatcgca cttttggtcc aggacggctt cctcgacctc       240

gacgccgaag tggtcaagta ctggccggaa ttcggcgccg aaggaaaggc cacgattacc       300

gtggcccagc tgctctccca ccaggccggg cttctgggag tcgaaggcgg actcaccctc       360

gcggaataca acaactccga actggccgcc gccaagctcg cgcagatgcg gccgctgtgg       420

aagcccggga ccgccttcgg gtaccacgcc ctgaccatcg gcgtcttcat ggaggagctt 480

tgccgccgga tcaccgggtc cacgctccag gaaatctacg aacagcggat ccgctcggtc 540

acgggcgccc acttcttcct gggactgcct gagtccgagg aaccccgcta tgccaccctc 600

cgttgggctg cagacccctc ccagccgtgg attgatcccg ccagccattt cggcctttcc 660

gcaaactcgg ccgtggggga catccttgac ctgcccaacc tccgcgaggt ccgcgcagcc 720

ggcctgagtt cagccgccgg agtcgccagc gcggaaggca tggcccgcgt ctacgctgcg 780

gcactcaccg gacttgccgc caacggcgac cgagccgccg tcgcgcccct cctcagcgaa 840

gagaccatcc aaaccgtcac ggccgagcag gtcttcggca tcgaccgggt gttcggcgag 900

acgagctgct ttgggacagt gttcatgaaa tcgcatgcac gctcgcctta tggcagctac 960

cgggcgttcg ggcacgacgg cgccagcgca tctttggggt tcgctgaccc tgtgtatgaa 1020

ctcgccttcg ggtacgtgcc gcaacaggcc gagccgggcg gagcgggatg ccgcaacctt 1080

gagctgagcg ccgccgtgcg gaaggcagtc accgaactgg ctcagtag 1128

<210> 13
<211> 1128
<212> DNA
<213> Artificial Sequence

<220>
<223> Mutant A328G

<400> 13
gtggatgcac agacgattgc ccctggattc gaatcagtcg ccgaactctt tggccgtttc 60

ctgagcgaag accgggaata ttcagcccag ctcgcggcct accaccgcgg agtcaaggta 120

ttggacatca gcggtgggcc gcaccgccgc ccggattccg tgaccggtgt tttctcctgc 180

tccaagggag tatccgggct ggtcatcgca cttttggtcc aggacggctt cctcgacctc 240

gacgccgaag tggtcaagta ctggccggaa ttcggcgccg aaggaaaggc cacgattacc 300

gtggcccagc tgctctccca ccaggccggg cttctgggag tcgaaggcgg actcaccctc 360

gcggaataca acaactccga actggccgcc gccaagctcg cgcagatgcg gccgctgtgg 420

aagcccggga ccgccttcgg gtaccacgcc ctgaccatcg gcgtcttcat ggaggagctt 480

tgccgccgga tcaccgggtc cacgctccag gaaatctacg aacagcggat ccgctcggtc 540

acgggcgccc acttcttcct gggactgcct gagtccgagg aaccccgcta tgccaccctc 600

cgttgggctg cagacccctc ccagccgtgg attgatcccg ccagccattt cggcctttcc 660

gcaaactcgg ccgtggggga catccttgac ctgcccaacc tccgcgaggt ccgcgcagcc 720

ggcctgagtt cagccgccgg agtcgccagc gcggaaggca tggcccgcgt ctacgctgcg 780

gcactcaccg gacttgccgc caacggcgac cgagccgccg tcgcgcccct cctcagcgaa 840

gagaccatcc aaaccgtcac ggccgagcag gtcttcggca tcgaccgggt gttcggcgag 900

acgagctgct ttgggacagt gttcatgaaa tcgcatgcac gctcgcctta tggcagctac 960

cgggcgttcg ggcacgacgg cgggagcgca tctttggggt tcgctgaccc tgtgtatgaa 1020

ctcgccttcg ggtacgtgcc gcaacaggcc gagccgggcg gagcgggatg ccgcaacctt 1080

gagctgagcg ccgccgtgcg gaaggcagtc accgaactgg ctcagtag 1128

<210> 14
<211> 1128
<212> DNA
<213> Artificial Sequence

<220>
<223> Mutant A328G/F217W

<400> 14
gtggatgcac agacgattgc ccctggattc gaatcagtcg ccgaactctt tggccgtttc 60

ctgagcgaag accgggaata ttcagcccag ctcgcggcct accaccgcgg agtcaaggta 120

ttggacatca gcggtgggcc gcaccgccgc ccggattccg tgaccggtgt tttctcctgc 180

tccaagggag tatccgggct ggtcatcgca cttttggtcc aggacggctt cctcgacctc 240

gacgccgaag tggtcaagta ctggccggaa ttcggcgccg aaggaaaggc cacgattacc 300

gtggcccagc tgctctccca ccaggccggg cttctgggag tcgaaggcgg actcaccctc 360

gcggaataca acaactccga actggccgcc gccaagctcg cgcagatgcg gccgctgtgg 420

aagcccggga ccgccttcgg gtaccacgcc ctgaccatcg gcgtcttcat ggaggagctt 480

tgccgccgga tcaccgggtc cacgctccag gaaatctacg aacagcggat ccgctcggtc 540

acgggcgccc acttcttcct gggactgcct gagtccgagg aaccccgcta tgccaccctc 600

cgttgggctg cagacccctc ccagccgtgg attgatcccg ccagccattg ggcctttccc 660

gtgaactcgg ccgtggggga catccttgac ctgcccaacc tccgcgaggt ccgcgcagcc 720

ggcctgagtt cagccgccgg agtcgccagc gcggaaggca tggcccgcgt ctacgctgcg 780

gcactcaccg gacttgccgc caacggcgac cgagccgccg tcgcgcccct cctcagcgaa 840

gagaccatcc aaaccgtcac ggccgagcag gtcttcggca tcgaccgggt gttcggcgag 900

acgagctgct ttgggacagt gttcatgaaa tcgcatgcac gctcgcctta tggcagctac 960

cgggcgttcg ggcacgacgg cgggagcgca tctttggggt tcgctgaccc tgtgtatgaa 1020

ctcgccttcg ggtacgtgcc gcaacaggcc gagccgggcg gagcgggatg ccgcaacctt 1080

gagctgagcg ccgccgtgcg gaaggcagtc accgaactgg ctcagtag 1128

<210> 15
<211> 1128
<212> DNA
<213> Artificial Sequence

<220>
<223>  Mutant A328G/A221F

<400>  15
gtggatgcac agacgattgc ccctggattc gaatcagtcg ccgaactctt tggccgtttc      60

ctgagcgaag accgggaata ttcagcccag ctcgcggcct accaccgcgg agtcaaggta     120

ttggacatca gcggtgggcc gcaccgccgc ccggattccg tgaccggtgt tttctcctgc     180

tccaagggag tatccgggct ggtcatcgca cttttggtcc aggacggctt cctcgacctc     240

gacgccgaag tggtcaagta ctggccggaa ttcggcgccg aaggaaaggc cacgattacc     300

gtggcccagc tgctctccca ccaggccggg cttctgggag tcgaaggcgg actcaccctc     360

gcggaataca acaactccga actggccgcc gccaagctcg cgcagatgcg gccgctgtgg     420

aagcccggga ccgccttcgg gtaccacgcc ctgaccatcg gcgtcttcat ggaggagctt     480

tgccgccgga tcaccgggtc cacgctccag gaaatctacg aacagcggat ccgctcggtc     540

acgggcgccc acttcttcct gggactgcct gagtccgagg aaccccgcta tgccaccctc     600

cgttgggctg cagacccctc ccagccgtgg attgatcccg ccagccattt cggcctttcc     660

tttaactcgg ccgtggggga catccttgac ctgcccaacc tccgcgaggt ccgcgcagcc     720

ggcctgagtt cagccgccgg agtcgccagc gcggaaggca tggcccgcgt ctacgctgcg     780

gcactcaccg gacttgccgc caacggcgac cgagccgccg tcgcgcccct cctcagcgaa     840

gagaccatcc aaaccgtcac ggccgagcag gtcttcggca tcgaccgggt gttcggcgag     900

acgagctgct ttgggacagt gttcatgaaa tcgcatgcac gctcgcctta tggcagctac     960

cgggcgttcg ggcacgacgg cgggagcgca tctttggggt tcgctgaccc tgtgtatgaa    1020

ctcgccttcg ggtacgtgcc gcaacaggcc gagccgggcg gagcgggatg ccgcaacctt    1080

gagctgagcg ccgccgtgcg gaaggcagtc accgaactgg ctcagtag                 1128

<210>  16
<211>  1128
<212>  DNA
<213>  Artificial Sequence

<220>
<223>  Mutant A328G/A221M

<400>  16
gtggatgcac agacgattgc ccctggattc gaatcagtcg ccgaactctt tggccgtttc      60

ctgagcgaag accgggaata ttcagcccag ctcgcggcct accaccgcgg agtcaaggta     120

ttggacatca gcggtgggcc gcaccgccgc ccggattccg tgaccggtgt tttctcctgc     180

tccaagggag tatccgggct ggtcatcgca cttttggtcc aggacggctt cctcgacctc     240

gacgccgaag tggtcaagta ctggccggaa ttcggcgccg aaggaaaggc cacgattacc     300

gtggcccagc tgctctccca ccaggccggg cttctgggag tcgaaggcgg actcaccctc 360

gcggaataca acaactccga actggccgcc gccaagctcg cgcagatgcg gccgctgtgg 420

aagcccggga ccgccttcgg gtaccacgcc ctgaccatcg gcgtcttcat ggaggagctt 480

tgccgccgga tcaccgggtc cacgctccag gaaatctacg aacagcggat ccgctcggtc 540

acgggcgccc acttcttcct gggactgcct gagtccgagg aaccccgcta tgccaccctc 600

cgttgggctg cagacccctc ccagccgtgg attgatcccg ccagccattt cggcctttcc 660

atgaactcgg ccgtggggga catccttgac ctgcccaacc tccgcgaggt ccgcgcagcc 720

ggcctgagtt cagccgccgg agtcgccagc gcggaaggca tggcccgcgt ctacgctgcg 780

gcactcaccg gacttgccgc caacggcgac cgagccgccg tcgcgcccct cctcagcgaa 840

gagaccatcc aaaccgtcac ggccgagcag gtcttcggca tcgaccgggt gttcggcgag 900

acgagctgct ttgggacagt gttcatgaaa tcgcatgcac gctcgcctta tggcagctac 960

cgggcgttcg ggcacgacgg cgggagcgca tctttggggt tcgctgaccc tgtgtatgaa 1020

ctcgccttcg ggtacgtgcc gcaacaggcc gagccgggcg gagcgggatg ccgcaacctt 1080

gagctgagcg ccgccgtgcg gaaggcagtc accgaactgg ctcagtag 1128

<210> 17
<211> 1128
<212> DNA
<213> Artificial Sequence

<220>
<223> Mutant A328G/A221L

<400> 17
gtggatgcac agacgattgc ccctggattc gaatcagtcg ccgaactctt tggccgtttc 60

ctgagcgaag accgggaata ttcagcccag ctcgcggcct accaccgcgg agtcaaggta 120

ttggacatca gcggtgggcc gcaccgccgc ccggattccg tgaccggtgt tttctcctgc 180

tccaagggag tatccgggct ggtcatcgca cttttggtcc aggacggctt cctcgacctc 240

gacgccgaag tggtcaagta ctggccggaa ttcggcgccg aaggaaaggc cacgattacc 300

gtggcccagc tgctctccca ccaggccggg cttctgggag tcgaaggcgg actcaccctc 360

gcggaataca acaactccga actggccgcc gccaagctcg cgcagatgcg gccgctgtgg 420

aagcccggga ccgccttcgg gtaccacgcc ctgaccatcg gcgtcttcat ggaggagctt 480

tgccgccgga tcaccgggtc cacgctccag gaaatctacg aacagcggat ccgctcggtc 540

acgggcgccc acttcttcct gggactgcct gagtccgagg aaccccgcta tgccaccctc 600

cgttgggctg cagacccctc ccagccgtgg attgatcccg ccagccattt cggcctttcc 660

ctgaactcgg ccgtggggga catccttgac ctgcccaacc tccgcgaggt ccgcgcagcc 720

ggcctgagtt cagccgccgg agtcgccagc gcggaaggca tggcccgcgt ctacgctgcg 780

gcactcaccg gacttgccgc caacggcgac cgagccgccg tcgcgcccct cctcagcgaa 840

gagaccatcc aaaccgtcac ggccgagcag gtcttcggca tcgaccgggt gttcggcgag 900

acgagctgct ttgggacagt gttcatgaaa tcgcatgcac gctcgcctta tggcagctac 960

cgggcgttcg ggcacgacgg cgggagcgca tctttggggt tcgctgaccc tgtgtatgaa 1020

ctcgccttcg ggtacgtgcc gcaacaggcc gagccgggcg gagcgggatg ccgcaacctt 1080

gagctgagcg ccgccgtgcg gaaggcagtc accgaactgg ctcagtag 1128

<210> 18
<211> 1128
<212> DNA
<213> Artificial Sequence

<220>
<223> Mutant A328G/A221V

<400> 18
gtggatgcac agacgattgc ccctggattc gaatcagtcg ccgaactctt tggccgtttc 60

ctgagcgaag accgggaata ttcagcccag ctcgcggcct accaccgcgg agtcaaggta 120

ttggacatca gcggtgggcc gcaccgccgc ccggattccg tgaccggtgt tttctcctgc 180

tccaagggag tatccgggct ggtcatcgca cttttggtcc aggacggctt cctcgacctc 240

gacgccgaag tggtcaagta ctggccggaa ttcggcgccg aaggaaaggc cacgattacc 300

gtggcccagc tgctctccca ccaggccggg cttctgggag tcgaaggcgg actcaccctc 360

gcggaataca acaactccga actggccgcc gccaagctcg cgcagatgcg gccgctgtgg 420

aagcccggga ccgccttcgg gtaccacgcc ctgaccatcg gcgtcttcat ggaggagctt 480

tgccgccgga tcaccgggtc cacgctccag gaaatctacg aacagcggat ccgctcggtc 540

acgggcgccc acttcttcct gggactgcct gagtccgagg aaccccgcta tgccaccctc 600

cgttgggctg cagacccctc ccagccgtgg attgatcccg ccagccattt cggcctttcc 660

gtgaactcgg ccgtggggga catccttgac ctgcccaacc tccgcgaggt ccgcgcagcc 720

ggcctgagtt cagccgccgg agtcgccagc gcggaaggca tggcccgcgt ctacgctgcg 780

gcactcaccg gacttgccgc caacggcgac cgagccgccg tcgcgcccct cctcagcgaa 840

gagaccatcc aaaccgtcac ggccgagcag gtcttcggca tcgaccgggt gttcggcgag 900

acgagctgct ttgggacagt gttcatgaaa tcgcatgcac gctcgcctta tggcagctac 960

cgggcgttcg ggcacgacgg cgggagcgca tctttggggt tcgctgaccc tgtgtatgaa 1020

ctcgccttcg ggtacgtgcc gcaacaggcc gagccgggcg gagcgggatg ccgcaacctt 1080

gagctgagcg ccgccgtgcg gaaggcagtc accgaactgg ctcagtag 1128

<210> 19

<211>  1128
<212>  DNA
<213>  Artificial Sequence

<220>
<223>  Mutant A328G/I228L

<400>  19
gtggatgcac agacgattgc ccctggattc gaatcagtcg ccgaactctt tggccgtttc      60

ctgagcgaag accgggaata ttcagcccag ctcgcggcct accaccgcgg agtcaaggta     120

ttggacatca gcggtgggcc gcaccgccgc ccggattccg tgaccggtgt tttctcctgc     180

tccaagggag tatccgggct ggtcatcgca cttttggtcc aggacggctt cctcgacctc     240

gacgccgaag tggtcaagta ctggccggaa ttcggcgccg aaggaaaggc cacgattacc     300

gtggcccagc tgctctccca ccaggccggg cttctgggag tcgaaggcgg actcaccctc     360

gcggaataca acaactccga actggccgcc gccaagctcg cgcagatgcg gccgctgtgg     420

aagcccggga ccgccttcgg gtaccacgcc ctgaccatcg gcgtcttcat ggaggagctt     480

tgccgccgga tcaccgggtc cacgctccag gaaatctacg aacagcggat ccgctcggtc     540

acgggcgccc acttcttcct gggactgcct gagtccgagg aaccccgcta tgccaccctc     600

cgttgggctg cagacccctc ccagccgtgg attgatcccg ccagccattt cggcctttcc     660

gtgaactcgg ccgtggggga cctgcttgac ctgcccaacc tccgcgaggt ccgcgcagcc     720

ggcctgagtt cagccgccgg agtcgccagc gcggaaggca tggcccgcgt ctacgctgcg     780

gcactcaccg gacttgccgc caacggcgac cgagccgccg tcgcgcccct cctcagcgaa     840

gagaccatcc aaaccgtcac ggccgagcag gtcttcggca tcgaccgggt gttcggcgag     900

acgagctgct ttgggacagt gttcatgaaa tcgcatgcac gctcgcctta tggcagctac     960

cgggcgttcg ggcacgacgg cgggagcgca tctttggggt tcgctgaccc tgtgtatgaa    1020

ctcgccttcg ggtacgtgcc gcaacaggcc gagccgggcg gagcgggatg ccgcaacctt    1080

gagctgagcg ccgccgtgcg gaaggcagtc accgaactgg ctcagtag                 1128


<210>  20
<211>  1128
<212>  DNA
<213>  Artificial Sequence

<220>
<223>  Mutant A328G/A221F/D326V

<400>  20
gtggatgcac agacgattgc ccctggattc gaatcagtcg ccgaactctt tggccgtttc      60

ctgagcgaag accgggaata ttcagcccag ctcgcggcct accaccgcgg agtcaaggta     120

ttggacatca gcggtgggcc gcaccgccgc ccggattccg tgaccggtgt tttctcctgc     180

tccaagggag tatccgggct ggtcatcgca cttttggtcc aggacggctt cctcgacctc     240

gacgccgaag tggtcaagta ctggccggaa ttcggcgccg aaggaaaggc cacgattacc 300

gtggcccagc tgctctccca ccaggccggg cttctgggag tcgaaggcgg actcaccctc 360

gcggaataca acaactccga actggccgcc gccaagctcg cgcagatgcg gccgctgtgg 420

aagcccggga ccgccttcgg gtaccacgcc ctgaccatcg gcgtcttcat ggaggagctt 480

tgccgccgga tcaccgggtc cacgctccag gaaatctacg aacagcggat ccgctcggtc 540

acgggcgccc acttcttcct gggactgcct gagtccgagg aaccccgcta tgccaccctc 600

cgttgggctg cagacccctc ccagccgtgg attgatcccg ccagccattt cggcctttcc 660

tttaactcgg ccgtggggga catccttgac ctgcccaacc tccgcgaggt ccgcgcagcc 720

ggcctgagtt cagccgccgg agtcgccagc gcggaaggca tggcccgcgt ctacgctgcg 780

gcactcaccg gacttgccgc caacggcgac cgagccgccg tcgcgcccct cctcagcgaa 840

gagaccatcc aaaccgtcac ggccgagcag gtcttcggca tcgaccgggt gttcggcgag 900

acgagctgct ttgggacagt gttcatgaaa tcgcatgcac gctcgcctta tggcagctac 960

cgggcgttcg ggcacgtcgg cgggagcgca tctttggggt tcgctgaccc tgtgtatgaa 1020

ctcgccttcg ggtacgtgcc gcaacaggcc gagccgggcg gagcgggatg ccgcaacctt 1080

gagctgagcg ccgccgtgcg gaaggcagtc accgaactgg ctcagtag 1128

<210> 21
<211> 1128
<212> DNA
<213> Artificial Sequence

<220>
<223> Mutant A328G/A221F/D326V/F298L

<400> 21
gtggatgcac agacgattgc ccctggattc gaatcagtcg ccgaactctt tggccgtttc 60

ctgagcgaag accgggaata ttcagcccag ctcgcggcct accaccgcgg agtcaaggta 120

ttggacatca gcggtgggcc gcaccgccgc ccggattccg tgaccggtgt tttctcctgc 180

tccaagggag tatccgggct ggtcatcgca cttttggtcc aggacggctt cctcgacctc 240

gacgccgaag tggtcaagta ctggccggaa ttcggcgccg aaggaaaggc cacgattacc 300

gtggcccagc tgctctccca ccaggccggg cttctgggag tcgaaggcgg actcaccctc 360

gcggaataca acaactccga actggccgcc gccaagctcg cgcagatgcg gccgctgtgg 420

aagcccggga ccgccttcgg gtaccacgcc ctgaccatcg gcgtcttcat ggaggagctt 480

tgccgccgga tcaccgggtc cacgctccag gaaatctacg aacagcggat ccgctcggtc 540

acgggcgccc acttcttcct gggactgcct gagtccgagg aaccccgcta tgccaccctc 600

cgttgggctg cagacccctc ccagccgtgg attgatcccg ccagccattt cggcctttcc 660

tttaactcgg ccgtggggga catccttgac ctgcccaacc tccgcgaggt ccgcgcagcc 720

ggcctgagtt cagccgccgg agtcgccagc gcggaaggca tggcccgcgt ctacgctgcg 780

gcactcaccg gacttgccgc caacggcgac cgagccgccg tcgcgcccct cctcagcgaa 840

gagaccatcc aaaccgtcac ggccgagcag gtcttcggca tcgaccgggt gctcggcgag 900

acgagctgct ttgggacagt gttcatgaaa tcgcatgcac gctcgcctta tggcagctac 960

cgggcgttcg ggcacgtcgg cgggagcgca tctttggggt tcgctgaccc tgtgtatgaa 1020

ctcgccttcg ggtacgtgcc gcaacaggcc gagccgggcg gagcgggatg ccgcaacctt 1080

gagctgagcg ccgccgtgcg gaaggcagtc accgaactgg ctcagtag 1128

<210> 22
<211> 1128
<212> DNA
<213> Artificial Sequence

<220>
<223> Mutant A328G/A221F/D326V/F298L/N222D

<400> 22
gtggatgcac agacgattgc ccctggattc gaatcagtcg ccgaactctt tggccgtttc 60

ctgagcgaag accgggaata ttcagcccag ctcgcggcct accaccgcgg agtcaaggta 120

ttggacatca gcggtgggcc gcaccgccgc ccggattccg tgaccggtgt tttctcctgc 180

tccaagggag tatccgggct ggtcatcgca cttttggtcc aggacggctt cctcgacctc 240

gacgccgaag tggtcaagta ctggccggaa ttcggcgccg aaggaaaggc cacgattacc 300

gtggcccagc tgctctccca ccaggccggg cttctgggag tcgaaggcgg actcaccctc 360

gcggaataca acaactccga actggccgcc gccaagctcg cgcagatgcg gccgctgtgg 420

aagcccggga ccgccttcgg gtaccacgcc ctgaccatcg gcgtcttcat ggaggagctt 480

tgccgccgga tcaccgggtc cacgctccag gaaatctacg aacagcggat ccgctcggtc 540

acgggcgccc acttcttcct gggactgcct gagtccgagg aaccccgcta tgccaccctc 600

cgttgggctg cagacccctc ccagccgtgg attgatcccg ccagccattt cggcctttcc 660

tttgactcgg ccgtggggga catccttgac ctgcccaacc tccgcgaggt ccgcgcagcc 720

ggcctgagtt cagccgccgg agtcgccagc gcggaaggca tggcccgcgt ctacgctgcg 780

gcactcaccg gacttgccgc caacggcgac cgagccgccg tcgcgcccct cctcagcgaa 840

gagaccatcc aaaccgtcac ggccgagcag gtcttcggca tcgaccgggt gctcggcgag 900

acgagctgct ttgggacagt gttcatgaaa tcgcatgcac gctcgcctta tggcagctac 960

cgggcgttcg ggcacgtcgg cgggagcgca tctttggggt tcgctgaccc tgtgtatgaa 1020

ctcgccttcg ggtacgtgcc gcaacaggcc gagccgggcg gagcgggatg ccgcaacctt 1080

gagctgagcg ccgccgtgcg gaaggcagtc accgaactgg ctcagtag 1128

<210>  23
<211>  372
<212>  PRT
<213>  Arthrobacter sp. Rue61a

<400>  23

Met His Ser Gln Val Ile Ala Pro Gly Phe Glu Pro Val Ala Glu Leu
1               5                   10                  15

Phe Gly Val Phe Leu Glu Gln Asp Pro Asp Tyr Ser Ala Gln Val Ala
            20                  25                  30

Ala Tyr His Arg Gly Val Lys Val Leu Asp Leu Ser Gly Gly Pro His
        35                  40                  45

Ile Arg Pro Asp Ser Val Thr Gly Val Phe Ser Cys Ser Lys Gly Met
    50                  55                  60

Ala Gly Leu Val Met Ala Leu Leu Val Gln Asp Gly Glu Leu Asp Leu
65                  70                  75                  80

Glu Ala Glu Val Val Lys Tyr Trp Pro Glu Phe Gly Val Glu Gly Lys
                85                  90                  95

Ser Ser Ile Thr Val Ala Gln Leu Leu Ser His Arg Ala Gly Leu Leu
            100                 105                 110

Gly Val Glu Gly Gly Leu Thr Leu His Glu Val Asn Asn Ser Glu Leu
        115                 120                 125

Ala Ala Ala Lys Leu Ala Glu Leu Pro Pro Leu Trp Lys Pro Gly Thr
    130                 135                 140

Ala Phe Gly Tyr His Ala Leu Thr Ile Gly Ile Phe Met Glu Glu Leu
145                 150                 155                 160

Cys Arg Arg Ile Thr Gly Ser Thr Leu Gln Glu Val Phe Glu Gln Arg
                165                 170                 175

Ile Arg Ala Val Thr Gly Ala Asn Phe Tyr Leu Gly Leu Pro Glu Ser
            180                 185                 190

Glu Glu Ser Arg Phe Ala Gln Phe Arg Trp Ala Ala Asp Pro Ser Trp
        195                 200                 205

Pro Trp Val Asp Pro Ala Ser His Phe Gly Leu Ala Ala Asn Ala Ala
    210                 215                 220

Val Gly Asp Ile Leu Asp Leu Pro Asn Ile Arg Glu Val Arg Ala Ala
225                 230                 235                 240

Gly Leu Ser Ser Ala Ala Gly Val Ala Ser Ala Glu Gly Met Ala Arg
                245                 250                 255

Ile Tyr Ala Ala Ala Leu Thr Gly Leu Glu Gly Lys Ser Ala Met Pro
            260                 265                 270

Pro Leu Leu Thr Glu Glu Thr Ile Arg Thr Val Ser Ala Glu Gln Val
        275                 280                 285

Phe Gly Ile Asp Arg Val Phe Gly Glu Thr Gly Cys Phe Gly Thr Val
    290                 295                 300

Phe Met Lys Ser His Thr Arg Met Pro Phe Gly Ser Tyr Arg Ala Phe
305                 310                 315                 320

Gly His Asp Gly Ala Ser Ala Ser Leu Gly Phe Ala Asp Pro Val Tyr
                325                 330                 335

Glu Leu Gly Phe Gly Tyr Val Pro Gln Thr Ala Glu Pro Gly Gly Val
            340                 345                 350

Gly Cys Arg Asn Phe Gln Leu Ser Ser Ala Val Arg Glu Val Ile Ala
        355                 360                 365

Gly Phe Ala Ala
    370

## Description

### [Technical Field]

The present invention relates to modification of an enzyme showing hydrolysis activity with ethyl chrysanthemate (chrysanthemic acid esterase). Specifically, the present invention relates to modified chrysanthemic acid esterase with altered stereoselectivity and use thereof. This application claims priority to Japanese Patent Application No. 2018-229479 filed on December 6, 2018, which is incorporated herein in its entirety by reference.

### [Background Art]

Chrysanthemic acid is widely used as a synthesis starting material of synthetic pyrethroid insecticides. Chrysanthemic acid has 4 kinds of isomers ((1R,3R)-chrysanthemic acid, (1R,3S)-chrysanthemic acid, (1S,3S)-chrysanthemic acid and (1S,3R)-chrysanthemic acid), and (1R,3R)-chrysanthemic acid shows the highest insecticidal activity. Industrially, chrysanthemic acid is produced using a chemical synthesis method. On the other hand, chrysanthemic acid is also produced by an enzymatic reaction by using chrysanthemic acid esterase. Past reports on chrysanthemic acid esterase are shown below (patent document 1, non-patent documents 1 - 3). Patent document 1 describes that Arthrobacter globiformis-derived esterase degrades chrysanthemic acid ethylester in a 1R,3R((+)-trans)-specific manner (Table 1). In addition, non-patent document 1 describes that Arthrobacter globiformis-derived esterase and esterase of other origin degrade chrysanthemic acid ethylester in a 1R,3R((+)-trans)-specific or 1S,3R((-)-trans)-specific manner. Non-patent document 2 describes that, as the insecticidal effect of each isomer of chrysanthemic acid, 1R,3R((+)-trans) and 1R,3S((+)-cis) show different insecticidal effects. Non-patent document 3 is a research report on the degradation of chrysanthemic acid ethylester by Arthrobacter globiformis-derived esterase, and shows that the stereoselectivity changes as a result of a specific mutation (K62R mutation) (reactivity with (1S,3R)-ethyl chrysanthemate is exhibited).

### [Document List]

### [Patent document]

patent document 1: JP-A-H5-56787

### [Non-patent documents]

non-patent document 1: Agric. Biol. Chem., 55(11), 2865-2870, 1991
non-patent document 2: Chemistry and Actions of the Recent Synthetic Pyrethroids and their Stereoisomers, Hirosuke Yoshioka, Synthetic Organic Chemistry, Vol. 38, No. 12 (1980), 1151-1162
non-patent document 3: Production of optically active intermediate of pyrethroid insecticides by a microbial enzyme, Materials Science, Nara Institute of Science and Technology, Masako Nishizawa, March 2011

### [Summary of Invention]

### [Technical Problem]

One of the isomers of chrysanthemic acid, (1R,3S)-chrysanthemic acid, also has insecticidal activity and is useful in itself. An enzyme that efficiently produces (1R,3S)-chrysanthemic acid (i.e., esterase with high selectivity for (1R, 3S)-ethyl chrysanthemate) has not been known, and (1R,3S)-chrysanthemic acid is currently obtained by chemical hydrolysis of a mixture of ethyl chrysanthemate isomers, followed by optical resolution (purification using chiral column). From the aspects of yield and cost, or effluent regulation, it is desirable to produce (1R,3S)-chrysanthemic acid by using an enzyme; however, no enzyme has been reported that acts selectively or specifically for (1R,3S)-ethyl chrysanthemate. An object of the present invention is to provide an esterase useful for the production of (1R,3S)-chrysanthemic acid.

### [Solution to Problem]

To solve the above-mentioned problems, the present inventors attempted to modify the stereoselectivity of esterase derived from Arthrobacter globiformis by a protein engineering method. The esterase is specific to (1R,3R)-ethyl chrysanthemate, and cannot be used in practice for the production of (1R,3S)-chrysanthemic acid. After trial and error, they succeeded in identifying a plurality of mutation points (amino acid residues) that are effective in improving reactivity or selectivity for (1R,3S)-ethyl chrysanthemate, and obtained highly useful variants (modified esterases). Particularly, a variant (A328G/A221F) in which two amino acids are substituted is an enzyme that acts specifically on (1R,3S)-ethyl chrysanthemate, and its usefulness and utility value are extremely high. The mutation A328G, which is also contained in the variant, is an important mutation that can be said to define the stereoselectivity of the substrate.

The following invention is based on the above results and considerations.
[1] A modified esterase comprising an amino acid sequence resulting from an amino acid substitution of any one of the following (1) to (23) in the amino acid sequence of SEQ ID NO: 1, or an amino acid sequence having not less than 90% identity with the amino acid sequence (wherein the amino acid sequences are different in a portion other than the position of the aforementioned amino acid substitution), the modified esterase having improved reactivity with (1R,3S)-ethyl chrysanthemate as compared to an esterase consisting of the amino acid sequence of SEQ ID NO: 1:
   (1) mutation point: F58, amino acid after substitution: E, I, C, A, M or Y;
   (2) mutation point: D227, amino acid after substitution: I, S, A, W, H, G, F or V;
   (3) mutation point: 1228, amino acid after substitution: Y, S, T or W;
   (4) mutation point: L229, amino acid after substitution: Y, F or V;
   (5) mutation point: L231, amino acid after substitution: M, A or G;
   (6) mutation point: S244, amino acid after substitution: F;
   (7) mutation point: A245, amino acid after substitution: N or G;
   (8) mutation point: V297, amino acid after substitution: R, W, Q, A or I;
   (9) mutation point: F298, amino acid after substitution: S;
   (10) mutation point: A328, amino acid after substitution: N;
   (11) mutation point: A328, amino acid after substitution: G;
   (12) mutation points: A328 and F58, amino acid at mutation point A328 after substitution: G, amino acid at mutation point F58 after substitution: L or E;
   (13) mutation points: A328 and L151, amino acid at mutation point A328 after substitution: G, amino acid at mutation point L151 after substitution: Q, R, H, W, A, V, M, N, C, T or I;
   (14) mutation points: A328 and F217, amino acid at mutation point A328 after substitution: G, amino acid at mutation point F217 after substitution: W, G, A, D, T, R, E, V or Y;
   (15) mutation points: A328 and S220, amino acid at mutation point A328 after substitution: G, amino acid at mutation point S220 after substitution: Q, L, N or G;
   (16) mutation points: A328 and A221, amino acid at mutation point A328 after substitution: G, amino acid at mutation point A221 after substitution: F, M, L, V, E, R, G or S;
   (17) mutation points: A328 and D227, amino acid at mutation point A328 after substitution: G, amino acid at mutation point D227 after substitution: E or C;
   (18) mutation points: A328 and 1228, amino acid at mutation point A328 after substitution: G, amino acid at mutation point 1228 after substitution: L or G;
   (19) mutation points: A328 and L229, amino acid at mutation point A328 after substitution: G, amino acid at mutation point L229 after substitution: R, V, I or H;
   (20) mutation points: A328 and V297, amino acid at mutation point A328 after substitution: G, amino acid at mutation point V297 after substitution: C or L;
   (21) mutation points: A328, A221, and D326, amino acid at mutation point A328 after substitution: G, amino acid at mutation point A221 after substitution: F, amino acid at mutation point D326 after substitution: V;
   (22) mutation points: A328, A221, D326, and F298, amino acid at mutation point A328 after substitution: G, amino acid at mutation point A221 after substitution: F, amino acid at mutation point D326 after substitution: V, an amino acid at mutation point F298 after substitution: L;
   (23) mutation points A328, A221, D326, F298, and N222, amino acid at mutation point A328 after substitution: G, amino acid at mutation point A221 after substitution: F, amino acid at mutation point D326 after substitution: V, amino acid at mutation point F298 after substitution: L, amino acid at mutation point N222 after substitution: D.
[2] The modified esterase of [1], wherein the amino acid after substitution in (1) is E, I, C, A or M,
   the amino acid after substitution in (2) is I, S, A, W, H or G,
   the amino acid after substitution in (3) is Y or S,
   the amino acid after substitution in (4) is Y or F,
   the amino acid after substitution in (5) is M or A,
   the amino acid after substitution in (8) is R, W or Q,
   the amino acid at mutation point F58 after substitution in (12) is L,
   the amino acid at mutation point L151 after substitution in (13) is Q or R,
   the amino acid at mutation point F217 after substitution in (14) is W, G, A, D, T, R or E,
   the amino acid at mutation point S220 after substitution in (15) is Q or L,
   the amino acid at mutation point A221 after substitution in (16) is F, M, L, V, E, R or G,
   the amino acid at mutation point L229 after substitution in (19) is R, V or I.
[3] The modified esterase of [1], wherein the amino acid after substitution in (4) is Y,
   the amino acid after substitution in (5) is M,
   the amino acid after substitution in (8) is R or W,
   the amino acid at mutation point L151 after substitution in (13) is Q,
   the amino acid at mutation point F217 after substitution in (14) is W, G, A, D or T,
   the amino acid at mutation point A221 after substitution in (16) is F, M, L, V, E or R.
[4] The modified esterase of [1], wherein the amino acid substitution is any one of (11) to (23), and the modified esterase has improved selectivity for (1R,3S)-ethyl chrysanthemate.
[5] The modified esterase of [4], wherein the amino acid substitution is A328G, A328G/F217W, A328G/A221M, A328G/A221L, A328G/A221V, or A328G/I228L.
[6] The modified esterase of [4], wherein the amino acid substitution is A328G/A221F, and the modified esterase acts specifically for (1R,3S)-ethyl chrysanthemate.
[7] The modified esterase of [4], wherein the amino acid substitution is A328G/A221F/D326V.
[8] The modified esterase of [4], wherein the amino acid substitution is A328G/A221F/D326V/F298L.
[9] The modified esterase of [4], wherein the amino acid substitution is A328G/A221F/D326V/F298L/N222D.
[10] The modified esterase of any one of [1] to [9], wherein the aforementioned identity is not less than 95%.
[11] The modified esterase of [1], consisting of the amino acid sequence of any of SEQ ID NOs: 2 to 11.
[12] A gene encoding the modified esterase of any one of [1] to [11] .
[13] The gene of [12], comprising the base sequence of any of SEQ ID NOs: 13 to 22.
[14] A recombinant DNA comprising the gene of [12] or [13].
[15] A microorganism harboring the recombinant DNA of [14].
[16] An enzyme preparation comprising the modified esterase of any one of [1] to [11].
[17] A method for preparing a modified esterase, comprising the following steps (I) to (III):
   (I) a step of preparing a nucleic acid encoding an amino acid sequence of the modified esterase of any one of [1] to [11] ;
   (II) a step of expressing the aforementioned nucleic acid, and
   (III) a step of collecting an expression product.
[18] The method of [17], wherein the aforementioned amino acid sequence is an amino acid sequence of any one of SEQ ID NOs: 2 to 11.
[19] The method of [17], wherein the aforementioned nucleic acid comprises a base sequence of any of SEQ ID NOs: 13 to 22.
[20] A method for producing (1R,3S)-chrysanthemic acid, comprising reacting the modified esterase of any one of [1] to [11] with (1R,3S)-ethyl chrysanthemate.
[21] The method of [20], wherein the (1R,3S)-ethyl chrysanthemate coexists with (1R,3R)-ethyl chrysanthemate, (1S,3S)-ethyl chrysanthemate and (1S,3R)-ethyl chrysanthemate.

### [Brief Description of Drawings]

Fig. 1 shows evaluation results of mutation F58X. The "reactivity with (1R,3S)-ethyl chrysanthemate" was evaluated by a value (B/A) obtained by dividing "ratio (%) of production amount of (1R,3S)-chrysanthemic acid to that of wild-type enzyme in reaction using (1R,3S)-ethyl chrysanthemate (sole substrate)" (B) by "ratio (%) of production amount of (1R,3R)-chrysanthemic acid to that of wild-type enzyme in a reaction using a mixed substrate" (A) (same for the following mutations).
Fig. 2 shows evaluation results of mutations F217X, S220X, D227X.
Fig. 3 shows evaluation results of mutations I228X, L229X, L231X.
Fig. 4 shows evaluation results of mutations S244X, A245X, V297X.
Fig. 5 shows evaluation results of mutations F298X, A328X.

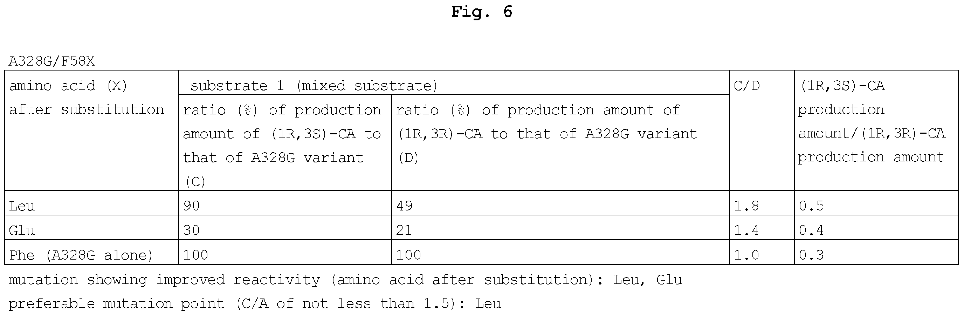

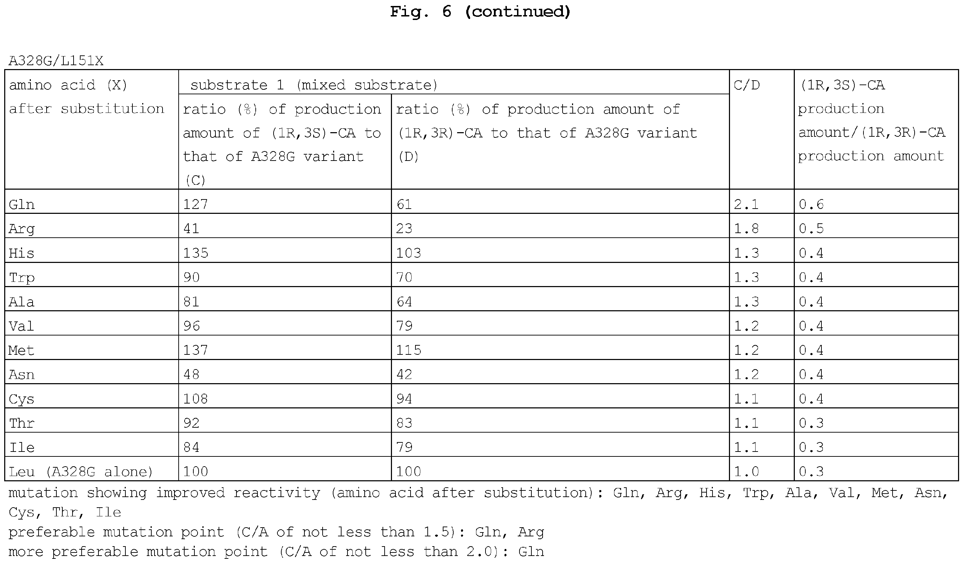

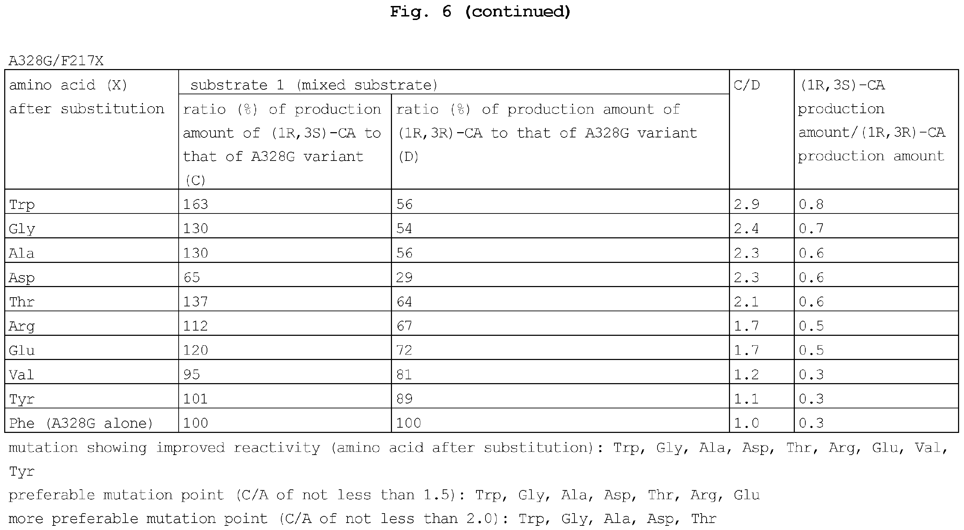

Fig. 6 shows evaluation results of combinatorial mutations A328G/F58X, A328G/L151X, A328G/F217X. The "selectivity for (1R,3S)-ethyl chrysanthemate" was evaluated by a value (C/D) obtained by dividing "ratio (%) of production amount of (1R,3S)-chrysanthemic acid to that of A328G variant in a reaction using a mixed substrate" (C) by "ratio (%) of production amount of (1R,3R)-chrysanthemic acid to that of A328G variant in a reaction using a mixed substrate" (D) (same for the following combinatorial mutations). The value of production amount of (1R,3S)-chrysanthemic acid/production amount of (1R,3R)-chrysanthemic acid is also shown.
Fig. 7 shows evaluation results of combinatorial mutations A328G/S220X, A328G/A221X, A328G/D227X.
Fig. 8 shows evaluation results of combinatorial mutations A328G/I228X, A328G/L229X, A328G/V297X.
Fig. 9 shows evaluation results of combinatorial mutations A328G/A221F, A328G/A221F/D326V, A328G/A221F/D326V/F298L, A328G/A221F/D326V/F298L/N222D.

### [Description of Embodiments]

For convenience of explanation, some of the terms used in relation to the present invention are defined in the following.

### (terms)

The term "modified esterase" refers to an enzyme obtained by modifying or mutating an esterase to be the standard (hereinafter to be referred to as "standard esterase"). The standard esterase is typically an esterase derived from a microorganism of the genus Arthrobacter (Arthrobacter globiformis) and having the amino acid sequence of SEQ ID NO: 1.

In the present invention, "substitution of amino acid" is performed as modification or mutation. Therefore, some of the amino acid residues are different when a modified esterase is compared with the standard esterase. In the present specification, a modified esterase is also referred to as a modified enzyme or variant.

In the present specification, each amino acid is denoted by a single letter as follows in a conventional manner.
methionine: M, serine: S, alanine: A, threonine: T, valine: V, tyrosine: Y, leucine: L, asparagine: N, isoleucine: I, glutamine: Q, proline: P, aspartic acid: D, phenylalanine: F, glutamic acid: E, tryptophan: W, lysine: K, cysteine: C, arginine: R, glycine: G, histidine: H

In the present specification, the position of a mutation point is specified by the number assigned from the N-terminal toward the C-terminal, with the amino acid residue on the N-terminal as the first amino acid residue.

In the present specification, the mutation by amino acid substitution is expressed by a combination of one letter representing an amino acid residue before substitution, a number representing a mutation point (position of an amino acid residue where amino acid substitution occurs), and one letter representing an amino acid residue after substitution. For example, a mutation in which alanine at position 328 is substituted by glycine is expressed as "A328G". In addition, when a combination of two mutations (combined use) is expressed, a symbol "/" is used. For example, a combination of a mutation in which alanine at position 328 is substituted by glycine and a mutation in which alanine at position 221 is substituted by phenylalanine is expressed as "A328G/A221F".

### 1. Modified esterase

The first aspect of the present invention relates to a modified esterase (modified enzyme). The modified enzyme of the present invention typically has an amino acid sequence comprising one, or two or more specific amino acid substitutions (mutations) in the amino acid sequence shown in SEQ ID NO: 1. Due to the characteristics, the reactivity with or selectivity for (1R,3S)-ethyl chrysanthemate or both are improved as compared with an esterase consisting of the amino acid sequence of SEQ ID NO: 1. The amino acid sequence of SEQ ID NO: 1 is the sequence of the Arthrobacter globiformis-derived esterase.

The "reactivity with (1R,3S)-ethyl chrysanthemate" is an expression also including the reactivity (relative reactivity) when compared with the reactivity with optical isomers of (1R,3S)-ethyl chrysanthemate ((1R,3R)-ethyl chrysanthemate, (1S,3S)-ethyl chrysanthemate, (1S,3R)-ethyl chrysanthemate). Therefore, the "improvement in the reactivity with (1R,3S)-ethyl chrysanthemate" is achieved by improvement in the selectivity for (1R,3S)-ethyl chrysanthemate, an increase in activity with (1R,3S)-ethyl chrysanthemate, a decrease in activity with optical isomers of (1R,3S)-ethyl chrysanthemate ((1R,3R)-ethyl chrysanthemate, (1S,3S)-ethyl chrysanthemate, (1S,3R)-ethyl chrysanthemate), and the like.

Arthrobacter globiformis-derived esterase (wild-type enzyme) has high selectivity for (1R,3R)-ethyl chrysanthemate. When 4 kinds of optical isomers ((1R,3R)-ethyl chrysanthemate, (1R,3S)-ethyl chrysanthemate, (1S,3S)-ethyl chrysanthemate, (1S,3R)-ethyl chrysanthemate) coexist, it preferentially hydrolyzes (1R,3R)-ethyl chrysanthemate. Therefore, only (1R,3R)-chrysanthemic acid is generally detected as a product. The wild-type enzyme also has a hydrolysis activity with (1R,3S)-ethyl chrysanthemate. However, since the reaction rate thereof is overwhelmingly slow compared to the reaction rate of the hydrolysis activity with (1R,3R)-chrysanthemic acid, it does not apparently react with (1R,3S)-ethyl chrysanthemate in the coexistence of (1R,3R)-ethyl chrysanthemate. On the other hand, when (1R,3S)-ethyl chrysanthemate is reacted alone as a substrate, (1R,3S)-chrysanthemic acid is detected as a resultant product thereof.

In the present application, considering the above-mentioned property of the wild-type enzyme, the "reactivity with (1R,3S)-ethyl chrysanthemate" is evaluated based on the amount of (1R,3S)-chrysanthemic acid produced in the reaction using only (1R,3S)-ethyl chrysanthemate (sole substrate) as the substrate, and the amount of (1R,3R)-ethyl chrysanthemate produced in the reaction using a mixed substrate of a mixture of (1R,3R)-ethyl chrysanthemate, (1R,3S)-ethyl chrysanthemate, (1S,3S)-ethyl chrysanthemate and (1S,3R)-ethyl chrysanthemate. Specifically, the "reactivity with (1R,3S)-ethyl chrysanthemate" is evaluated by a value obtained by dividing "ratio (%) of production amount of (1R,3S)-chrysanthemic acid to that of wild-type enzyme in reaction using (1R,3S)-ethyl chrysanthemate (sole substrate)" by "ratio (%) of production amount of (1R,3R)-chrysanthemic acid to that of wild-type enzyme in a reaction using a mixed substrate". When the value exceeds 1, it is judged that the "reactivity with (1R,3S)-ethyl chrysanthemate" has been improved. For a preferred modified enzyme, the value is not less than 2.0 and the degree of improvement in reactivity is high. For a more preferred modified enzyme, the value is not less than 3.0 and the reactivity has been further improved. The wild-type enzyme is an enzyme before modification, that is, the standard esterase.

As described above, the wild-type enzyme has high selectivity for (1R,3R)-ethyl chrysanthemate and (1R,3S)-chrysanthemic acid is not detected (is not produced substantially) when the wild-type enzyme is reacted with a mixed substrate. On the other hand, as shown in the below-mentioned Example, a variant in which amino acid alanine at position 328 of a wild-type enzyme was substituted by glycine (A328G variant) showed improved selectivity for (1R,3S)-ethyl chrysanthemate, showed hydrolysis activity with (1R,3S)-ethyl chrysanthemate even when it was reacted with a mixed substrate, and produced (1R,3S)-chrysanthemic acid. In the present application, therefore, the A328G variant is used for the evaluation of "selectivity for (1R,3S)-ethyl chrysanthemate". Specifically, the "selectivity for (1R,3S)-ethyl chrysanthemate" is evaluated by a value obtained by dividing "ratio (%) of production amount of (1R,3S)-chrysanthemic acid to that of A328G variant in a reaction using a mixed substrate" by "ratio (%) of the production amount of (1R,3R)-chrysanthemic acid to that of A328G variant in a reaction using a mixed substrate". When the value is one or more, it is judged that the selectivity is equal to or not less than that of A328G variant and the "selectivity for (1R,3S)-ethyl chrysanthemate" has been improved. For a preferred modified enzyme, the value is not less than 1.5 and the degree of improvement in selectivity is high. For a more preferred modified enzyme, the value is not less than 2.0 and the selectivity has been further improved. When the production amount of (1R,3R)-chrysanthemic acid is below detection limit, namely, when (1R,3R)-chrysanthemic acid is not substantially produced, the "ratio (%) of production amount of (1R,3S)-chrysanthemic acid to that of A328G variant in a reaction using a mixed substrate" is zero and the above-mentioned value cannot be calculated. A modified enzyme that produces such result (A328G/A221F variant is one example) can be said to be (1R,3S)-ethyl chrysanthemate specific.

In the present specification, "containing an amino acid substitution" means that an amino acid residue after substitution is located at a mutation point (i.e., position of an amino acid residue where specific amino acid substitution occurs). Therefore, when an amino acid sequence containing an amino acid substitution (mutated amino acid sequence) is compared with the amino acid sequence of SEQ ID NO: 1 not containing an amino acid substitution (standard amino acid sequence), the amino acid residue is different at the position of the amino acid substitution.

Amino acid substitutions (mutation points and amino acids after substitution) that are contained in the modified enzyme of the present invention and improve reactivity with (1R,3S)-ethyl chrysanthemate are recited below. Note that (11) to (23) are more preferred since they also improve selectivity for (1R,3S)-ethyl chrysanthemate in addition to reactivity with (1R,3S)-ethyl chrysanthemate.

(1) Mutation point is F58, an amino acid after substitution is E, I, C, A, M or Y. Preferably, an amino acid after substitution is E, I, C, A or M.
(2) Mutation point is D227, an amino acid after substitution is I, S, A, W, H, G, F or V. Preferably, an amino acid after substitution is I, S, A, W, H or G. Further preferably, an amino acid after substitution is I, S, A, W or H.
(3) Mutation point is 1228, an amino acid after substitution is Y, S, T or W. Preferably, an amino acid after substitution is Y or S.
(4) Mutation point is L229, an amino acid after substitution is Y, F or V. Preferably, an amino acid after substitution is Y or F. Further preferably, an amino acid after substitution is Y.
(5) Mutation point is L231, an amino acid after substitution is M, A or G. Preferably, an amino acid after substitution is M or A. Further preferably, an amino acid after substitution is M.
(6) Mutation point is S244, an amino acid after substitution is F.
(7) Mutation point is A245, an amino acid after substitution is N or G.
(8) Mutation point is V297, an amino acid after substitution is R, W, Q, A or I. Preferably, an amino acid after substitution is R, W or Q. Further preferably, an amino acid after substitution is R or W.
(9) Mutation point is F298, an amino acid after substitution is S.
(10) Mutation point is A328, an amino acid after substitution is N.
(11) Mutation point is A328, an amino acid after substitution is G.
(12) Mutation points are A328 and F58, an amino acid at mutation point A328 after substitution is G, an amino acid at mutation point F58 after substitution is L or E. Preferably, an amino acid at mutation point F58 after substitution is L.
(13) Mutation points are A328 and L151, an amino acid at mutation point A328 after substitution is G, an amino acid at mutation point L151 after substitution is Q, R, H, W, A, V, M, N, C, T or I. Preferably, an amino acid at mutation point L151 after substitution is Q or R. Further preferably, an amino acid at mutation point L151 after substitution is Q.
(14) Mutation points are A328 and F217, an amino acid at mutation point A328 after substitution is G, an amino acid at mutation point F217 after substitution is W, G, A, D, T, R, E, V or Y. Preferably, an amino acid at mutation point F217 after substitution is W, G, A, D, T, R or E. Further preferably, an amino acid at mutation point F217 after substitution is W, G, A, D or T.
(15) Mutation points are A328 and S220, an amino acid at mutation point A328 after substitution is G, an amino acid at mutation point S220 after substitution is Q, L, N or G. Preferably, an amino acid at mutation point S220 after substitution is Q or L.
(16) Mutation points are A328 and A221, an amino acid at mutation point A328 after substitution is G, an amino acid at mutation point A221 after substitution is F, M, L, V, E, R, G or S. Preferably, an amino acid at mutation point A221 after substitution is F, M, L, V, E, R or G. Further preferably, an amino acid at mutation point A221 after substitution is F, M, L, V, E or R.
(17) Mutation points are A328 and D227, an amino acid at mutation point A328 after substitution is G, an amino acid at mutation point D227 after substitution is E or C.
(18) Mutation points are A328 and 1228, an amino acid at mutation point A328 after substitution is G, an amino acid at mutation point 1228 after substitution is L or G.
(19) Mutation points are A328 and L229, an amino acid at mutation point A328 after substitution is G, an amino acid at mutation point L229 after substitution is R, V, I or H. Preferably, an amino acid at mutation point L229 after substitution is R, V or I.
(20) Mutation points are A328 and V297, an amino acid at mutation point A328 after substitution is G, an amino acid at mutation point V297 after substitution is C or L.
(21) Mutation points are A328 and A221 and D326, an amino acid at mutation point A328 after substitution is G, an amino acid at mutation point A221 after substitution is F, an amino acid at mutation point D326 after substitution is V.
(22) Mutation points are A328 and A221 and D326 and F298, an amino acid at mutation point A328 after substitution is G, an amino acid at mutation point A221 after substitution is F, an amino acid at mutation point D326 after substitution is V, an amino acid at mutation point F298 after substitution is L.
(23) Mutation points are A328 and A221 and D326 and F298 and N222, an amino acid at mutation point A328 after substitution is G, an amino acid at mutation point A221 after substitution is F, an amino acid at mutation point D326 after substitution is V, an amino acid at mutation point F298 after substitution is L, an amino acid at mutation point N222 after substitution is D.

As regards (14) and (15) in the above amino acid substitutions, the reactivity was not improved when mutation point F217 or mutation point S220 was used solely, whereas the selectivity for (1R,3S)-ethyl chrysanthemate was improved when each mutation point was used in combination with A328G mutation, which is noteworthy. In addition, when mutation points are A328 and A221, an amino acid at mutation point A328 after substitution is G, and an amino acid at mutation point A221 after substitution is F, the selectivity for (1R,3S)-ethyl chrysanthemate is strikingly improved. Thus, a modified enzyme that specifically acts on (1R,3S)-ethyl chrysanthemate can be prepared, and the amino acid substitution is extremely preferable.

As specific examples of the modified enzyme of the present invention, esterases consisting of the amino acid sequence of any of SEQ ID NOs: 2 to 11 (respectively corresponding to a variant containing the amino acid substitution A328G of (11), a variant containing A328G/F217W which is one of the amino acid substitutions of (14), a variant containing A328G/A221F which is one of the amino acid substitutions of (16), a variant containing A328G/A221M which is one of the amino acid substitutions of (16), a variant containing A328G/A221L which is one of the amino acid substitutions of (16), a variant containing A328G/A221V which is one of the amino acid substitutions of (16), a variant containing A328G/I228L which is one of the amino acid substitutions of (18), a variant containing A328G/A221F/D326V which is one of the amino acid substitutions of (21), a variant containing A328G/A221F/D326V/F298L which is one of the amino acid substitutions of (22), a variant containing A328G/A221F/D326V/F298L/N222D which is one of the amino acid substitutions of (23)) can be mentioned. As shown in the below-mentioned Example, these variants are particularly improved in the selectivity for (1R,3S)-ethyl chrysanthemate. Among these, A328G/A221F variant specifically acts on (1R,3S)-ethyl chrysanthemate, and has particularly high usefulness. Also, A328G/A221F/D326V variant, A328G/A221F/D326V/F298L variant, and A328G/A221F/D326V/F298L/N222D variant have very high reactivity in addition to the selectivity for (1R,3S)-ethyl chrysanthemate, and are extremely useful.

In general, when the amino acid sequence of a certain protein is partially mutated, the protein obtained after the mutation may have a function equivalent to that of the protein before the mutation. That is, it sometimes occurs that a mutation in the amino acid sequence of a protein does not substantially affect the function of the protein and the function is maintained before and after the mutation. On the other hand, when two proteins show high identity in the amino acid sequence, it is highly probable that the two proteins exhibit equivalent properties. In consideration of such common technical knowledge, an enzyme having an amino acid sequence that is not completely identical (i.e., 100% identity) to the amino acid sequence of the above-mentioned modified enzyme, namely, "the amino acid sequence containing an amino acid substitution of any of the above-mentioned (1) to (23) in the amino acid sequence of SEQ ID NO: 1 (specific examples of the amino acid sequence are those of SEQ ID NOs: 2 to 11)", but shows high identity therewith, and exhibiting a desired property change can be considered to be an enzyme substantially identical to the above-mentioned modified enzyme (substantially identical esterase). The identity here is not less than 60%, not less than 70%, not less than 80%, not less than 82%, not less than 85%, not less than 90%, not less than 93%, not less than 95%, 98%, or not less than 99%. A higher identity is more preferable. Therefore, in a most preferred embodiment, the identity is not less than 99%.

When the above-mentioned modified enzyme and a substantially identical esterase are compared, a slight difference in the amino acid sequence is found. The difference in the amino acid sequence occurs at a position other than the position at which the above-mentioned amino acid substitution is applied. Therefore, for example, when the standard of identity is the amino acid sequence of SEQ ID NO: 2, the difference in the amino acid sequence is found at a position other than G at position 328; when the standard of identity is the amino acid sequence of SEQ ID NO: 3, a position other than G at position 328 and W at position 217; when the standard of identity is the amino acid sequence of SEQ ID NO: 4, a position other than G at position 328 and F at position 221; when the standard of identity is the amino acid sequence of SEQ ID NO: 5, a position other than G at position 328 and M at position 221; when the standard of identity is the amino acid sequence of SEQ ID NO: 6, a position other than G at position 328 and L at position 221; when the standard of identity is the amino acid sequence of SEQ ID NO: 7, a position other than G at position 328 and V at position 221; when the standard of identity is the amino acid sequence of SEQ ID NO: 8, a position other than G at position 328 and L at position 228; when the standard of identity is the amino acid sequence of SEQ ID NO: 9, a position other than G at position 328, F at position 221, and V at position 326; when the standard of identity is the amino acid sequence of SEQ ID NO: 10, a position other than G at position 328, F at position 221, V at position 326, and L at position 298; when the standard of identity is the amino acid sequence of SEQ ID NO: 11, a position other than G at position 328, F at position 221, V at position 326, L at position 298, and D at position 222. In other words, in an amino acid sequence having the above-mentioned identity (not less than 60%, not less than 70%, not less than 80%, not less than 82%, not less than 85%, not less than 90%, not less than 93%, not less than 95%, 98%, or not less than 99%) with the amino acid sequence of SEQ ID NO: 2, the amino acid at position 328 is G. Similarly, in an amino acid sequence having the above-mentioned identity with the amino acid sequence of SEQ ID NO: 3, the amino acid at position 328 is G and the amino acid at position 217 is W; in an amino acid sequence having the above-mentioned identity with the amino acid sequence of SEQ ID NO: 4, the amino acid at position 328 is G and the amino acid at position 221 is F; in an amino acid sequence having the above-mentioned identity with the amino acid sequence of SEQ ID NO: 5, the amino acid at position 328 is G and the amino acid at position 221 is M; in an amino acid sequence having the above-mentioned identity with the amino acid sequence of SEQ ID NO: 6, the amino acid at position 328 is G and the amino acid at position 221 is L; in an amino acid sequence having the above-mentioned identity with the amino acid sequence of SEQ ID NO: 7, the amino acid at position 328 is G and the amino acid at position 221 is V; in an amino acid sequence having the above-mentioned identity with the amino acid sequence of SEQ ID NO: 8, the amino acid at position 328 is G and the amino acid at position 228 is L; in an amino acid sequence having the above-mentioned identity with the amino acid sequence of SEQ ID NO: 9, the amino acid at position 328 is G, the amino acid at position 221 is F, and the amino acid at position 326 is V; in an amino acid sequence having the above-mentioned identity with the amino acid sequence of SEQ ID NO: 10, the amino acid at position 328 is G, the amino acid at position 221 is F, the amino acid at position 326 is V, and the amino acid at position 298 is L; in an amino acid sequence having the above-mentioned identity with the amino acid sequence of SEQ ID NO: 11, the amino acid at position 328 is G, the amino acid at position 221 is F, the amino acid at position 326 is V, the amino acid at position 298 is L, and the amino acid at position 222 is D.

The "slight difference in the amino acid sequence" here results from deletion, substitution, addition or insertion of amino acids, or a combination thereof. It typically means that an amino acid sequence is mutated (or changed) by deletion or substitution of one to several (upper limit is, for example, 3, 5, 7, 10) amino acids constituting the amino acid sequence, or addition or insertion of one to several (upper limit is, for example, 3, 5, 7, 10) amino acids constituting the amino acid sequence, or a combination of these. The "slight difference in the amino acid sequence" preferably results from a conservative amino acid substitution. The "conservative amino acid substitution" here means substitution of a certain amino acid residue by an amino acid residue having a side chain with similar properties. Amino acid residues are classified into some families according to the side chains thereof, such as basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), β-branched side chains (e.g., threonine, valine, isoleucine), and aromatic side chains (e.g., tyrosine, phenylalanine, tryptophan, histidine). The conservative amino acid substitution is preferably a substitution between amino acid residues within the same family. It is known that the active residues of esterase derived from Arthrobacter globiformis (SEQ ID NO: 1) are the 59-position amino acid serine, the 325-position amino acid histidine, and the 326-position amino acid aspartic acid. When making a mutation, therefore, it is advisable not to affect the acid residues.

The identity (%) between two amino acid sequences or two base sequences (hereinafter "two sequences" is used as a term including these) can be determined, for example, by the following procedure. First, two sequences are aligned to allow for optimum comparison (for example, a gap may be introduced into the first sequence to optimize the alignment with the second sequence). When a molecule (amino acid residue or nucleotide) at a specific position in the first sequence is the same as a molecule at the corresponding position in the second sequence, the molecules at those positions can be determined as being identical. The identity between two sequences is a function of the number of identical positions common to the two sequences (i.e., identity (%) = number of identical positions/total number of positions × 100). Preferably, the number and size of the gaps required to optimize the alignment are also taken into consideration.

The comparison and determination of the identity between two sequences can be realized using a mathematical algorithm. Specific examples of the mathematical algorithm that can be used for comparison of the sequences include, but are not limited to, an algorithm described in Karlin and Altschul (1990) Proc. Natl. Acad. Sci. USA 87:2264-68 and modified by Karlin and Altschul (1993) Proc. Natl. Acad. Sci. USA 90:5873-77. Such algorithm is incorporated into NBLAST program and XBLAST program (version 2.0) described in Altschul et al. (1990) J. Mol. Biol. 215: 403-10. In order to obtain an equivalent nucleotide sequence, for example, BLAST nucleotide search may be performed using the NBLAST program with score = 100, wordlength = 12. In order to obtain an equivalent amino acid sequence, for example, BLAST polypeptide search may be performed using the XBLAST program with score = 50, wordlength = 3. In order to obtain gap alignment for comparison, Gapped BLAST described in Altschul et al. (1997) Amino Acids Research 25(17): 3389-3402 can be utilized. When using BLAST and Gapped BLAST, the default parameter of a corresponding program (e.g., XBLAST and NBLAST) can be used. For detail, see http://www.ncbi.nlm.nih.gov. Examples of other mathematical algorithm that can be used for comparison of sequences include the algorithm described in Meyers and Miller (1988) Comput. App1. Biosci. 4: 11-17. Such algorithms are incorporated into, for example, ALIGN program that is available from GENESTREAM network server (IGH Montpellier, France) or ISREC server. When the ALIGN program is utilized for comparison of amino acid sequences, for example, PAM120 residue mass table can be used with gap length penalty=12, gap penalty=4.

The identity between two amino acid sequences can be determined using the GAP program in the GCG software package, and using Blossom 62 matrix or PAM250 matrix with gap weighting=12, 10, 8, 6, or 4, gap length weighting=2, 3, or 4. In addition, the identity between two base sequences can be determined using the GAP program in the GCG software package (available at http://www.gcg.com) with gap weighting=50, gap length weighting=3.

Typically, an esterase consisting of the amino acid sequence of SEQ ID NO: 1, i.e., an esterase derived from Arthrobacter globiformis is mutated (amino acid substitution of any of the above-mentioned (1) to (23)) to afford the modified enzyme of the present invention. The above-mentioned substantially identical esterase can be obtained by applying a further mutation to one obtained by mutating an esterase consisting of the amino acid sequence of SEQ ID NO: 1 (amino acid substitution of any of the above-mentioned (1) to (23)), by applying an equivalent mutation to an esterase consisting of an amino acid sequence with high identity with the amino acid sequence of SEQ ID NO: 1, such as an esterase derived from the same species and genus as the Arthrobacter globiformis strain that produces an esterase consisting of the amino acid sequence of SEQ ID NO: 1, or the like, or by applying a further mutation to one obtained by the above-mentioned mutagenesis. By the "equivalent mutation" here, an amino acid residue corresponding to the amino acid residue at the mutation point according to the present invention (the mutation point in the amino acid substitution of any of the above-mentioned (1) to (23)) is substituted in an amino acid sequence with high identity with the amino acid sequence of SEQ ID NO: 1. Examples of the esterase consisting of an amino acid sequence with high identity with the amino acid sequence of SEQ ID NO: 1 include an esterase derived from Arthrobacter sp. Rue61a and having the amino acid sequence of SEQ ID NO: 23 (amino acid sequence identity of 81%).

In the present specification, the term "corresponding" when used for amino acid residues means equivalent contribution to the exhibition of functions between proteins (enzymes) being compared. For example, when an amino acid sequence to be compared with the amino acid sequence of standard esterase (the amino acid sequence of SEQ ID NO: 1) is aligned in consideration of partial homology of the primary structure (amino acid sequence) so that the optimal comparison can be achieved (where necessary, the alignment may be optimized by introducing a gap), an amino acid located at a position corresponding to a specific amino acid in the standard amino acid sequence can be specified as a "corresponding amino acid". The "corresponding amino acid" can also be specified by comparison between steric structures (three-dimensional structures) in place of or in addition to the comparison between primary structures. Utilizing the steric structure information, comparison results with high reliability can be obtained. In this case, a method of aligning while comparing atomic coordinates of the steric structures of a plurality of enzymes can be adopted. The steric structure information of an enzyme to be mutated can be obtained from, for example, the Protein Data Bank (http://www.pdbj.org/index_j.html).

One example of a method for determining a protein steric structure by the X-ray crystal structure analysis is shown below.
(1) A protein is crystallized. Crystallization is essential for steric structure determination. In addition, crystallization is also industrially useful as a purification method of a protein at high purity, and a high density and stable preservation method of a protein. In this case, it is preferable to crystallize a protein to which a substrate or an analogue compound thereof is bound as a ligand.
(2) The produced crystal is irradiated with X-ray and diffraction data is collected. In many cases, a protein crystal is damaged by X-ray irradiation and the diffraction ability is deteriorated. In such cases, a low temperature measurement technique in which the crystal is rapidly cooled to about -173°C and the diffraction data is collected in this state has recently been prevailing. In addition, finally, synchrotron radiation having high brilliance is utilized to collect high resolution data to be used for structural determination.
(3) Crystal structure analysis requires phase information in addition to the diffraction data. When the crystal structure of a protein analogous to a desired protein is unknown, it is impossible to determine the structure by a molecular replacement method, and a phase problem needs to be solved by a heavy-atom isomorphous replacement method. The heavy-atom isomorphous replacement method is a method including introducing a metallic atom having a high atomic number such as mercury, platinum and the like into a crystal and obtaining phase information by utilizing contribution of a large X-ray scattering ability of the metallic atom to X-ray diffraction data. The determined phase can be improved by smoothing the electron density of a solvent region in the crystal. Since a water molecule in the solvent region has large fluctuation, the electron density is hardly observed. By approximating the electron density in this region to 0, it is possible to approach the real electron density, which in turn improves the phase. When plural molecules are contained in an asymmetric unit, the phase is further improved significantly by averaging the electron density of these molecules. A protein model is fit to an electron density map calculated using the phase improved as described above. This process is performed on computer graphics by using a program such as QUANTA of MSI Co. (USA). Thereafter, structure refinement is performed using a program such as X-PLOR of MSI Co. to complete the structure analysis. When the crystal structure of a protein analogous to a desired protein is known, it can be determined by a molecule replacement method by using the atomic coordinate of a known protein. Molecular replacement and structure refinement can be performed using a program such as CNS_SOLVE ver. 11, and the like.

### 2. Nucleic acid encoding modified esterase, etc.

The second aspect of the present invention provides a nucleic acid relating to the modified enzyme of the present invention. That is, a gene encoding the modified enzyme, a nucleic acid that can be used as a probe for identifying a nucleic acid encoding the modified enzyme, and a nucleic acid that can be used as a primer for amplifying or mutating a nucleic acid encoding the modified enzyme are provided.

A gene encoding the modified enzyme is typically used in the preparation of the modified enzyme. According to a genetic engineering preparation method using a gene encoding the modified enzyme, a modified enzyme in a more homogeneous state can be obtained. In addition, the method can also be a method preferable for preparing a large amount of a modified enzyme. The use of the gene encoding a modified enzyme is not limited to preparation of a modified enzyme. For example, the nucleic acid can also be used as a tool for an experiment aiming at clarification of the action mechanisms of a modified enzyme or a tool for designing or producing a further modified form of an enzyme.

In the present specification, the "gene encoding a modified enzyme" refers to a nucleic acid capable of affording the modified enzyme when it is expressed, and includes not to mention a nucleic acid having a base sequence corresponding to the amino acid sequence of the modified enzyme, and a nucleic acid obtained by adding a sequence that does not encode an amino acid sequence to such a nucleic acid. In addition, the degeneracy of a codon is also taken into consideration.

Examples of the sequence (base sequence) of the gene encoding a modified enzyme are shown in SEQ ID NOs: 13 to 22. These sequences encode, as shown in the following, the variants described in the Examples set forth later.
SEQ ID NO: 13: A328G variant
SEQ ID NO: 14: A328G/A217W variant
SEQ ID NO: 15: A328G/A221F variant
SEQ ID NO: 16: A328G/A221M variant
SEQ ID NO: 17: A328G/A221L variant
SEQ ID NO: 18: A328G/A221V variant
SEQ ID NO: 19: A328G/I228L variant
SEQ ID NO: 20: A328G/A221F/D326V variant
SEQ ID NO: 21: A328G/A221F/D326V/F298L variant
SEQ ID NO: 22: A328G/A221F/D326V/F298L/N222D variant

When a gene according to the present invention is expressed in a host, a sequence encoding a signal peptide (signal sequence) may be added to the 5' terminal side of the above-mentioned sequence (any of SEQ ID NOs: 13 to 22). The signal sequence may be selected according to the host. Any signal sequence can be used in the present invention as long as it can express a variant of interest. Examples of the signal sequence that can be used in the present invention includes a sequence encoding the signal peptide of α-factor (Protein Engineering, 1996, vol. 9, p. 1055-1061), a sequence encoding the signal peptide of the α-factor receptor, a sequence encoding the signal peptide of SUC2 protein, a sequence encoding the signal peptide of PHO5 protein, a sequence encoding the signal peptide of BGL2 protein, a sequence encoding the signal peptide of AGA2 protein, a sequence encoding the signal peptide of TorA (trimethylamine N-oxide reductase), a sequence encoding the signal peptide of Bacillus subtilis-derived PhoD (phosphoesterase), a sequence encoding the signal peptide of Bacillus subtilis-derived LipA (lipase), a sequence encoding the signal peptide of Aspergillus oryzae-derived Taka-amylase (JP-A-2009-60804), a sequence encoding the signal peptide of Bacillus amyloliquefaciens-derived α-amylase (Eur. J. Biochem. 155, 577-581 (1986)), a sequence encoding the signal peptide of Bacillus subtilis-derived neutral protease (APPLIED AND ENVIRONMENTAL MICROBIOLOGY, Apr. 1995, p. 1610-1613, Vol. 61, No. 4), a sequence encoding the signal peptide of cellulase derived from a bacterium of the genus Bacillus (JP-A-2007-130012), and a sequence encoding pelB used for the pET system.

The nucleic acid of the present invention can be prepared in an isolated state by using a standard genetic engineering method, molecular biological method, biochemical method, chemical synthesis and the like by reference to the present specification or the sequence information disclosed in the appended Sequence Listing.

In another embodiment of the present invention, a nucleic acid partly different in the base sequence when compared with the base sequence of a gene encoding the modified enzyme of the invention, even though the functions of a protein encoded by the nucleic acid are equivalent is provided (hereinafter to be also referred to as a "homologous nucleic acid", and a base sequence defining the homologous nucleic acid is also referred to as a "homologous base sequence"). An example of the homologous nucleic acid is a DNA composed of a base sequence containing substitution, deletion, insertion, addition or inversion of 1 or plural bases in the base sequence of the nucleic acid encoding the modified enzyme of the present invention to be the standard, and encoding a protein having enzyme activity characteristic of a modified enzyme (i.e., esterase activity). The substitution, deletion and the like of the bases may occur in a plurality of sites. The "plurality" here varies depending on the positions and kinds of the amino acid residues in the steric structure of a protein encoded by the nucleic acid. It means, for example, 2 to 40 bases, preferably 2 to 20 bases, more preferably 2 to 10 bases.

The homologous nucleic acid has the identity of, for example, not less than 60%, preferably not less than 70%, more preferably not less than 80%, still more preferably not less than 85%, further preferably not less than about 90%, still more preferably not less than 95%, most preferably not less than 99%, with the base sequence to be the standard.

The homologous nucleic acid described above can be obtained by, for example, a restriction enzyme treatment, a treatment with exonuclease, DNA ligase, etc., introduction of mutation by a site-directed mutagenesis method (Molecular Cloning, Third Edition, Chapter 13, Cold Spring Harbor Laboratory Press, New York), or a random mutagenesis method (Molecular Cloning, Third Edition, Chapter 13, Cold Spring Harbor Laboratory Press, New York), and the like. The homologous nucleic acid can also be obtained by other methods such as exposure to ultraviolet radiation and the like.

Another embodiment of the present invention relates to a nucleic acid having a base sequence complementary to the base sequence of a gene encoding the modified enzyme of the invention. A still another embodiment of the present invention provides a nucleic acid having a base sequence with the identity of at least about 60%, 70%, 80%, 90%, 95%, 99%, or 99.9% with the base sequence of a gene encoding the modified enzyme of the present invention or a base sequence complementary to the base sequence.

A still another embodiment of the present invention relates to a nucleic acid having a base sequence that hybridizes under stringent conditions to a base sequence complementary to the base sequence of a gene encoding the modified enzyme of the present invention or a homologous base sequence thereof. The "stringent conditions" here refer to conditions under which a so-called specific hybrid is formed and a non-specific hybrid is not formed. Such stringent conditions are known to a person skilled in the art and can be set by reference to, for example, Molecular Cloning (Third Edition, Cold Spring Harbor Laboratory Press, New York) and Current protocols in molecular biology (edited by Frederick M. Ausubel et al., 1987). Examples of the stringent conditions include conditions of incubating using a hybridization solution (50% formamide, 10 × SSC (0.15 M NaCl, 15 mM sodium citrate, pH 7.0), a 5 × Denhardt solution, 1% SDS, 10% dextran sulfate, 10 µg/ml of denatured salmon sperm DNA, and a 50 mM phosphate buffer (pH7.5)) at about 42°C to about 50°C, and thereafter washing with 0.1 × SSC and 0.1% SDS at about 65°C to about 70°C. Examples of further preferable stringent conditions include conditions using 50% formamide, 5 × SSC (0.15 M NaCl, 15 mM sodium citrate, pH 7.0), a 1 × Denhardt solution, 1% SDS, 10% dextran sulfate, 10 µg/ml of denatured salmon sperm DNA, and a 50 mM phosphate buffer (pH 7.5) as a hybridization solution.

A still another embodiment of the present invention provides a nucleic acid (nucleic acid fragment) having a part of the base sequence of a gene encoding the modified enzyme of the invention or a base sequence complementary thereto. Such a nucleic acid fragment can be used in detection, identification, and/or amplification of a nucleic acid having the base sequence of a gene encoding the modified enzyme of the present invention, and the like. For example, the nucleic acid fragment is designed to contain at least a part that hybridizes to a continuous nucleotide part (for example, about 10 to about 100 bases in length, preferably about 20 to about 100 bases in length, further preferably about 30 to about 100 bases in length) in the base sequence of a gene encoding the modified enzyme of the invention. When used as a probe, the nucleic acid fragment can be labeled. For the labeling, for example, fluorescent substance, enzyme, and radioisotope can be used.

A still another aspect of the present invention relates to a recombinant DNA containing the gene of the present invention (gene encoding a modified enzyme). For example, the recombinant DNA of the invention is provided in the form of a vector. The term "vector" in the present specification refers to a nucleic acid molecule that can transport a nucleic acid inserted in the vector into a target such as a cell and the like.

A suitable vector is selected according to the object of use (cloning, expression of protein) and in consideration of the kind of the host cell. Examples of a vector for Escherichia coli as a host include pET21 or a modification thereof, M13 phage or a modification thereof, λ phage or a modification thereof, pBR322 or a modification thereof (e.g., pB325, pAT153, pUC8), and the like; examples of a vector for a yeast as a host include pYepSec1, pMFa, pYES2, pPIC3.5K, and the like; examples of a vector for an insect cell as a host include pAc, pVL, and the like; and examples of a vector for a mammalian cell as a host include pCDM8, pMT2PC, and the like.

The vector of the present invention is preferably an expression vector. The "expression vector" refers to a vector capable of introducing a nucleic acid inserted thereinto into a target cell (host cell) and expressing the nucleic acid in the cell. The expression vector generally contains a promoter sequence necessary for the expression of the inserted nucleic acid, an enhancer sequence that accelerates the expression, and the like. An expression vector containing a selection marker can also be used. When such expression vector is used, the presence or absence (and degree thereof) of introduction of the expression vector can be confirmed using a selection marker.

Insertion of the nucleic acid of the present invention into a vector, insertion of a selection marker gene (if necessary), insertion of a promoter (if necessary), and the like can be performed using a standard recombinant DNA technique (e.g., a well-known method using a restriction enzyme and a DNA ligase: Molecular Cloning, Third Edition, 1.84, Cold Spring Harbor Laboratory Press, New York can be referred to).

As a host cell, microorganisms such as koji mold (e.g., Aspergillus oryzae), bacterium of genus Bacillus (e.g., Bacillus subtilis), Escherichia coli, budding yeast (Saccharomyces cerevisiae) and the like can be used from the aspect of easiness of handling. Any host cell can be utilized as long as it permits replication of a recombinant DNA and expression of the gene of a modified enzyme. Preferably, Escherichia coli and Saccharomyces cerevisiae can be used. Examples of the Escherichia coli include Escherichia coli BL21(DE3) when a T7 series promoter is used, and Escherichia coli JM109 when a T7 series promoter is not used. Examples of Saccharomyces cerevisiae include Saccharomyces cerevisiae SHY2, Saccharomyces cerevisiae AH22, and Saccharomyces cerevisiae INVSc1 (Invitrogen).

Another aspect of the present invention relates to a microorganism harboring the recombinant DNA of the present invention (i.e., transformant). The microorganism of the present invention can be obtained by transfection or transformation using the above-mentioned vector of the present invention. For example, transfection or transformation can be performed by the calcium chloride method (J. Mol. Biol., 53, 159 (1970)), the Hanahan method (J. Mol. Biol., 166, 557 (1983)), the SEM method (Gene, 96, 23 (1990)), the method of Chung, et al. (Proc. Natl. Acad. Sci. U.S.A. 86, 2172 (1989)), the calcium phosphate coprecipitation method, electroporation (Potter, H. et al., Proc. Natl. Acad. Sci. U.S.A. 81, 7161-7165 (1984)), lipofectin (Felgner, P.L. et al., Proc. Natl. Acad. Sci. U.S.A. 84, 7413-7417 (1984)), and the like. The microorganism of the present invention can be used for producing the modified enzyme of the present invention.

### 3. Enzyme preparation containing modified esterase

The modified enzyme of the present invention is provided, for example, in the form of an enzyme preparation. The enzyme preparation may contain excipient, buffering agent, suspending agent, stabilizer, preservative, antiseptic, saline and the like besides the active ingredient (the modified enzyme of the present invention). As the excipient, starch, dextrin, maltose, trehalose, lactose, D-glucose, sorbitol, D-mannitol, sucrose, glycerol, and the like can be used. As the buffering agent, phosphate, citrate, acetate, and the like can be used. As the stabilizer, propylene glycol, ascorbic acid, and the like can be used. As the preservative, phenol, benzalkonium chloride, benzyl alcohol, chlorobutanol, methylparaben and the like can be used. As the antiseptic, ethanol, benzalkonium chloride, p-hydroxybenzoic acid, chlorobutanol and the like can be used.

### 4. Preparation method of modified esterase

A further aspect of the present invention relates to a method for preparing a modified enzyme. In one embodiment of the preparation method of the present invention, the modified enzyme that has been successfully obtained by the present inventors is prepared by a genetic engineering method. In this embodiment, a nucleic acid encoding the amino acid sequence of any of SEQ ID NOs: 2 to 11 is prepared (step (I)). As used herein, a "nucleic acid encoding a particular amino acid sequence" is a nucleic acid capable of affording a polypeptide having the amino acid sequence when it is expressed, and includes not to mention a nucleic acid consisting of a base sequence corresponding to the amino acid sequence. An extra sequence which may or may not encode an amino acid sequence may be added to such nucleic acid. In addition, the degeneracy of codon is also taken into consideration. The "nucleic acid encoding the amino acid sequence of any of SEQ ID NOs: 2 to 11" can be prepared in an isolated state by using a standard genetic engineering method, a molecular biological method, a biochemical method, and the like by reference to the present specification or the sequence information disclosed in the appended Sequence Listing. All of the amino acid sequences of SEQ ID NOs: 2 to 11 result from mutation of the amino acid sequence of an esterase derived from Arthrobacter globiformis. Therefore, a nucleic acid (gene) encoding the amino acid sequence of any of SEQ ID NOs: 2 to 11 can also be obtained by applying a required mutation to a gene encoding an esterase derived from Arthrobacter globiformis. Many methods for site-specific base sequence substitution are known in the art (see, for example, Molecular Cloning, Third Edition, Cold Spring Harbor Laboratory Press, New York), and an appropriate method can be selected and used from among those. As a site-specific mutagenesis method, a site-specific amino acid saturation mutagenesis method can be employed. The site-specific amino acid saturation mutagenesis method is a "Semi-rational, semi-random" method in which the position where a desired function of a protein is involved is assumed based on the steric structure thereof, and amino acid saturation mutation is introduced (J. Mol. Biol. 331, 585-592 (2003)). For example, site-specific amino acid saturation mutation can be introduced using a kit such as Quick change (Stratagene) or the like, and overlap extension PCR (Nucleic Acid Res. 16, 7351-7367 (1988)). As the DNA polymerase for use in the PCR, for example, Taq polymerase and the like can be used. It is preferable to use a DNA polymerase with high accuracy such as KOD-PLUS-(Toyobo Co., Ltd.), Pfu turbo (Stratagene) and the like.

Following step (I), the prepared nucleic acid is expressed (step (II)). For example, an expression vector with the above-mentioned nucleic acid inserted therein is prepared and a host cell is transformed using the vector.

Then, a transformant is cultured under the condition where a modified enzyme as an expression product is produced. The transformant may be cultured following a conventional method. Any assimilable carbon compound may be used as a carbon source to be used for a medium and, for example, glucose, sucrose, lactose, maltose, molasses, pyruvic acid and the like are used. The nitrogen source may be any utilizable nitrogen compound and, for example, peptone, meat extract, yeast extract, casein hydrolysate, soybean cake alkaline extract, and the like are used. In addition to these, salts such as phosphate, carbonate, sulfate, magnesium, calcium, potassium, iron, manganese, zinc and the like, specific amino acid, specific vitamin and the like are used as necessary.

On the other hand, the culture temperature can be set within the range of 30°C to 40°C (preferably around 37°C). The culture period can be set in consideration of the growth property of a transformant to be cultured and production property of the modified enzyme. The pH of the medium is adjusted to fall within the range where a transformant grows and an enzyme is produced. The pH of the medium is preferably set to about 6.0 to 9.0 (preferably around pH 7.0).

Subsequently, the expression product (modified enzyme) is collected (step (III)). A culture medium containing cells after culture may be used as an enzyme solution directly or after undergoing concentration, removal of impurities, and the like. In general, an expression product is once collected from the culture medium or cells. When the expression product is a secretory protein, it can be collected from the culture medium and, when otherwise, it can be collected from cells. When collecting from a culture medium, for example, a purified product of a modified enzyme can be obtained by removing insoluble materials by filtration and centrifugation of culture supernatant, and then performing separation and purification by a combination of concentration under reduced pressure, membrane concentration, salting out using ammonium sulfate or sodium sulfate, fractional precipitation method using methanol, ethanol, acetone or the like, dialysis, heat treatment, isoelectric point treatment, various kinds of chromatography such as gel filtration, adsorption chromatography, ion exchange chromatography, affinity chromatography and the like (e.g., gel filtration with Sephadex gel (GE Healthcare Bioscience), etc., DEAE Sepharose CL-6B (GE Healthcare Bioscience), octyl Sepharose CL-6B (GE Healthcare Bioscience), CM Sepharose CL-6B (GE Healthcare Bioscience)) and the like. On the other hand, when collecting from cells, a purified product of a modified enzyme can be obtained by subjecting the culture medium to filtration, centrifugation, or the like, to collect the cells, destroying the cells by a mechanical method such as pressurization, sonication, physical disruption treatment and the like, or an enzymatic method with lysozyme or the like, and performing separation and purification in the same manner as described above.

It is also possible to provide a purified enzyme obtained as described above after powderization by, for example, freeze drying, vacuum drying, spray drying or the like. In this case, the purified enzyme may be dissolved in advance in phosphate buffer, triethanolamine buffer, Tris-HCl buffer or GOOD buffer. Preferably, phosphate buffer or triethanolamine buffer can be used. As the GOOD buffer here, PIPES, MES or MOPS can be mentioned.

While the gene expression - recovery of expression product (modified enzyme) are generally performed using an appropriate host-vector system as described above, a cell-free synthesis system may also be employed. As used herein, the "cell-free synthesis system (cell-free transcription system, cell-free transcription/translation system)" refers to in vitro synthesis of mRNA or a protein from a nucleic acid (DNA or mRNA) as a template encoding the mRNA or the protein, without using living cells but using ribosome, transcription/translation factors and the like derived from living cells (or obtained by genetic engineering method). In the cell-free synthesis system, a cell extract obtained by purifying a cell homogenate as necessary is generally used. The cell extract generally contains a ribosome necessary for protein synthesis, various factors such as initiation factor, and the like, and various enzymes such as tRNA, and the like. When a protein is synthesized, other substances necessary for synthesizing a protein are added to the cell extract, such as various amino acids, energy sources (e.g., ATP, GTP, etc.), phosphocreatine, and the like. It is needless to say that ribosome, various factors and/or various enzymes, and the like, all of which prepared separately, may be supplemented as necessary in the protein synthesis.

The development of a transcription/translation system in which each molecule (factor) necessary for protein synthesis is reconstructed has also been reported (Shimizu, Y. et al.: Nature Biotech., 19, 751-755, 2001). In this synthesis system, genes for 31 kinds of factors constituting a bacterial protein synthesis system composed of 3 kinds of initiation factors, 4 kinds of factors involved in termination, 20 kinds of aminoacyl tRNA synthases that bind each amino acid to tRNA, and a methionyl tRNA formyltransferase are amplified from an Escherichia coli genome, and a protein synthesis system is reconstituted in vitro by using these. Such a reconstituted synthesis system may also be used in the present invention.

The term "cell-free transcription/translation system" is interchangeably used with a cell-free protein synthesis system, an in vitro translation system or an in vitro transcription/translation system. In the in vitro translation system, a protein is synthesized using RNA as a template. Any of total RNA, mRNA, an in vitro transcription product, or the like is used as the template RNA. In the other in vitro transcription/translation system, a DNA is used as the template. The template DNA should contain a ribosome bonding region, and preferably contains an appropriate terminator sequence. In the in vitro transcription/translation system, conditions including addition of factors necessary for each reaction are set to ensure continuous progress of a transcription reaction and a translation reaction.

### 5. Use of modified esterase

A further aspect of the present invention relates to the use of a modified enzyme, and provides a method for producing (1R,3S)-chrysanthemic acid by using the modified enzyme of the present invention. In the production method of the present invention, (1R,3S)-chrysanthemic acid is produced by hydrolyzing (1R,3S)-ethyl chrysanthemate by reaction with the modified enzyme of the present invention. The reaction conditions are not particularly limited as long as hydrolysis is achieved as desired. For example, the reaction can be performed using a buffer such as glycine-NaOH, PIPES or the like under conditions of pH 6 - 11, temperature 40°C - 60°C. As a substrate, (1R,3S)-ethyl chrysanthemate with high purity (e.g., purity of not less than 98%) may be used or a substrate in which (1R,3R)-ethyl chrysanthemate, (1S,3S)-ethyl chrysanthemate and (1S,3R)-ethyl chrysanthemate coexist (e.g., mixed ethyl chrysanthemate of
(1R,3R):(1S,3S):(1R,3S):(1S,3R)=3:3:2:2, etc.) may be used. The reaction product is optically resolved as necessary and used for various applications. For optical resolution, column chromatography can be used. An immobilized modified enzyme may also be used.

### [Example]

Wild-type esterase derived from Arthrobacter globiformis (amino acid sequence is shown in SEQ ID NO: 1 and gene sequence is shown in SEQ ID NO: 12) has high selectivity for (1R,3R)-ethyl chrysanthemate. An attempt was made to modify the stereoselectivity of the esterase by a protein engineering method.

### 1. Production of variant library

A variant library (CASTing Library and two-points combinatorial variant) was prepared by the following method.

### (1) Introduction of mutation and transformation

A PCR primer for mutagenesis was designed, and PCR was performed under the following conditions to introduce a mutation.

### <template plasmid>

pET21b (wild-type enzyme gene was introduced) about 30 ng/µL

### <PCR reaction composition> (total amount 20 µL)

5× Prime STAR GXL Buffer (Takara Bio Inc.): 4 µL
dNTP Mixture (2.5 mM each): 1.6 µL
template (about 30 ng/µL): 0.25 µL
F-primer: 0.2 µL
R-primer: 0.2 µL
Prime STAR GXL DNA Polymerase (Takara Bio Inc.): 0.8 µL

The above were mixed and adjusted to 20 µL with ultrapure water (MilliQ water)

### <PCR conditions>

reaction at 98°C for 1 min
20 cycles of reaction at 98°C for 10 sec, 60°C for 15 sec and 68°C for 2 min
reaction at 68°C for 5 min
allowed to stand at 4°C

The PCR reaction mixture (15 µL) was treated (37°C, 3 hr or more) with DpnI (1.5 µL). Using the DpnI-treated solution (2 µL), a ligation treatment (16°C, O/N) was performed with the following ligation reaction composition.

### <ligation reaction composition> (total amount 5.5 µL)

DpnI-treated PCR product: 2 µL
T4 Polynucleotide Kinase: 1 µL
Ligation High (Toyobo): 2.5 µL

Using the ligation reaction mixture (5.5 µL), E. coli BL21(DE3) (25 µL) was transformed (treated at 42°C for 45 sec and then rapidly cooled on ice). The transformation solution (about 30 µL) was spread on LB agar medium plate added with ampicillin (final concentration 0.1 mg/mL) and cultured at 37°C, O/N (preculture).

### (2) Obtainment of enzyme extract

Each mutant strain after preculture was inoculated into 1 mL of TB medium (invitrogen) added with ampicillin (final concentration 0.1 mg/mL). The cells were cultured at 33°C for 48 hr (IPTG (final concentration 0.1 mM) was added 24 hours after the start of the culture), and centrifuged (3,000 g × 10 min). The supernatant was removed and the cells were recovered. Then, the cells were lysed (25°C, 2 hr or more) with a lysis agent to extract the enzyme. The lysis suspension was centrifuged (3,000 g × 10 min), and the supernatant was recovered and used as an enzyme extract.

### (3) Production of combinatorial variant

Using A328G variant as a template, PCR was performed by an operation similar to that in the above-mentioned (1). The PCR product was recovered and an enzyme extract was obtained by an operation similar to that in the above-mentioned (2). A candidate mutation point to be combined with A328G was selected based on the evaluation results of CASTing Library and the results of docking simulation by MOE (MOLSIS).

### (4) Production of multiple variant

Using A328G/A221F variant as a template, random mutagenesis was performed by Error-prone PCR. Using trans-pNP chrysanthemic acid, screening was performed with the amount of liberated pNP as an index. Using the obtained variant as a template, similar mutagenesis and screening were repeated multiple times to produce a multiple variant showing an increased reactivity with (1R,3S)-ethyl chrysanthemate.

### 2. Evaluation of variant

### 2-1. Evaluation of CASTing Library

CASTing Library was evaluated by the following method. To evaluate changes in the reactivity with (1R,3S)-ethyl chrysanthemate, the following two kinds of substrates were used.
substrate 1 (mixed substrate): mixture of ethyl chrysanthemate (purity not less than 95%, (1R,3R) isomer, (1R,3S) isomer, (1S,3R) isomer, (1S,3S) isomer ((1R,3R):(1S,3S):(1R,3S):(1S,3R)=3:3:2:2), Tokyo Kagaku Kogyo)
substrate 2 (sole substrate): (1R,3S)-ethyl chrysanthemate

The substrate (substrate 1 or substrate 2) (1 µL) was dispensed to a reaction container, a measurement sample (enzyme extract prepared in the above-mentioned 1.(2)) (99 µL) was added and reacted (40°C, 24 hr). After the reaction, 6N HCl (10 µL) and hexane (150 µL) were added. After thorough stirring, the mixture was centrifuged and the organic layer (100 µL) was collected. The extract was dried to solidness, re-dissolved in an analysis solvent, and analyzed by supercritical fluid chromatography (SFC). The analysis conditions are shown below.

### <analysis conditions of supercritical fluid chromatograph (SFC) >

apparatus: ACQUITY UPC2 PDA system (Waters)
column: ACQUITY UPC2 Trefoil AMYl (Waters) 2.5 µm, 3.0×150 mm
mobile phase: CO₂/10 mM ammonium acetate-containing 2-propanol (0.5% acetic acid) = 92/8
flow: 2.0 mL/min
injection volume: 1 µL
analysis time: 1.5 min
PDA detector: 205-400 nm
resolution: 1.2 nm

Changes in the reactivity with (1R,3S)-ethyl chrysanthemate were evaluated based on the reactivity of a wild-type enzyme as the standard. To be specific, a value of "a ratio (%) of production amount of (1R,3S)-chrysanthemic acid to that of wild-type enzyme in a reaction using (1R,3S)-ethyl chrysanthemate (sole substrate)"/"a ratio (%) of production amount of (1R,3R)-chrysanthemic acid to that of wild-type enzyme in a reaction using a mixed substrate" was calculated from the SFC area. When the value exceeded 1, the reactivity with (1R,3S)-ethyl chrysanthemate was judged to have increased.

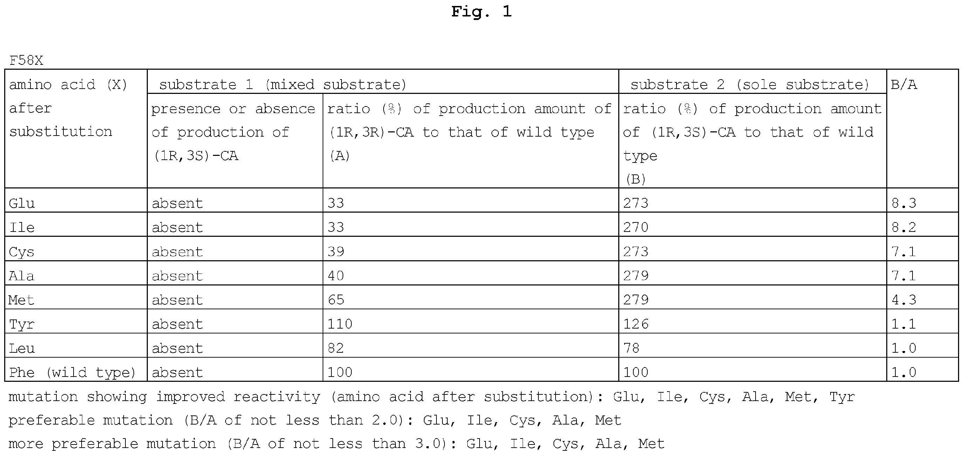

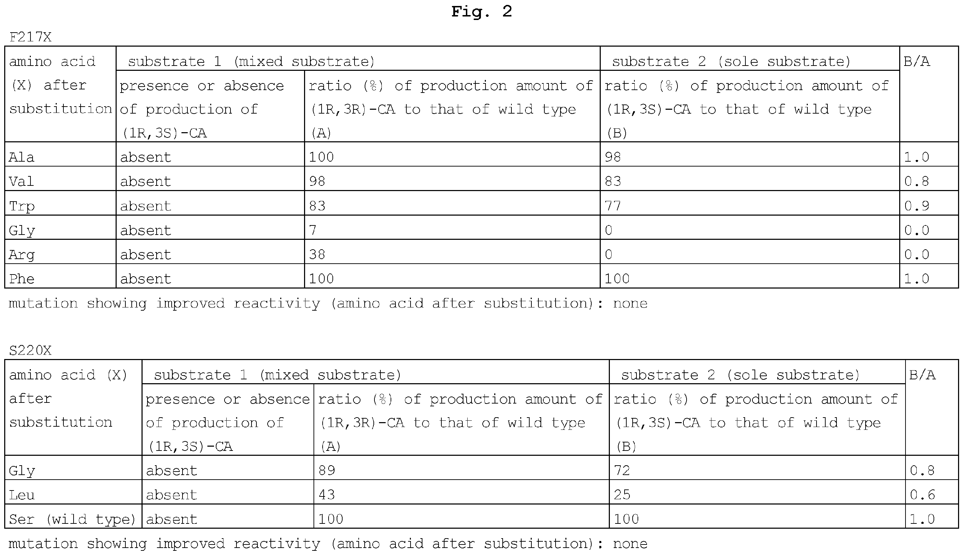

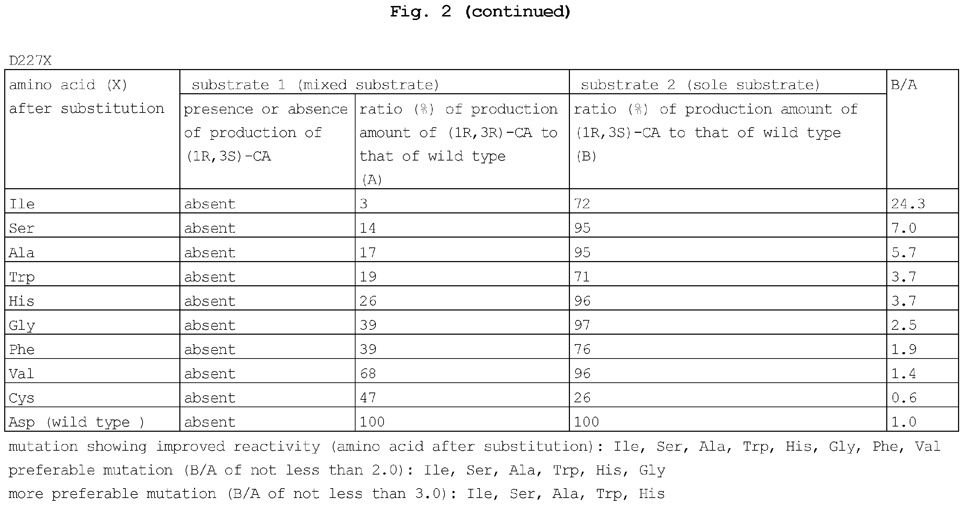

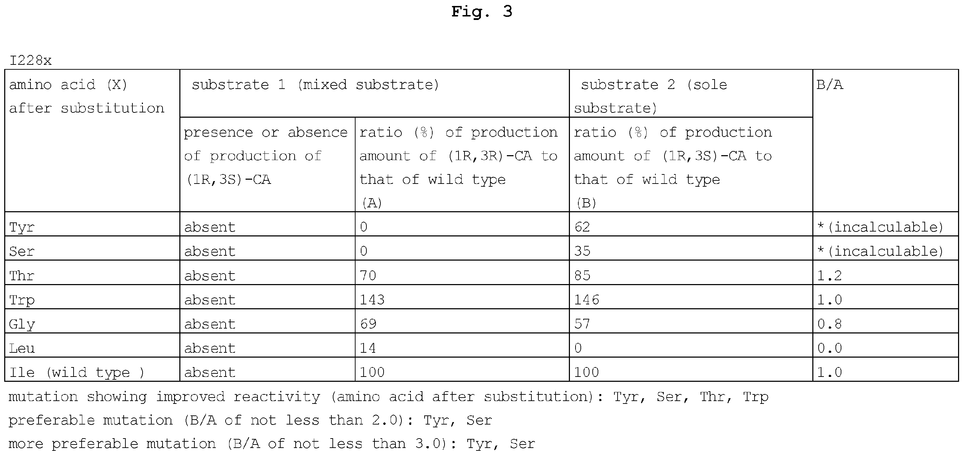

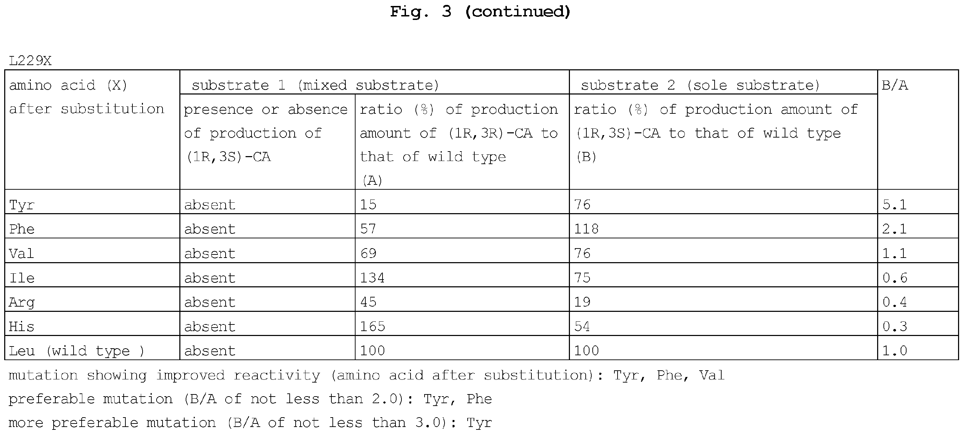

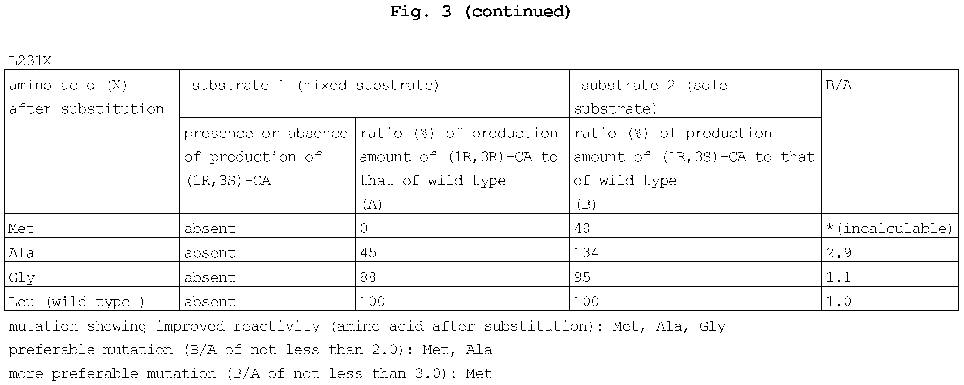

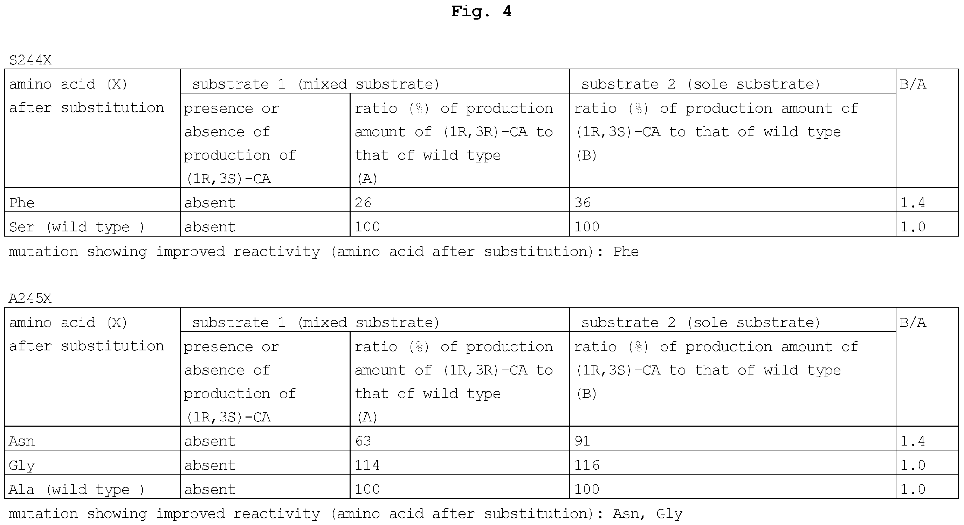

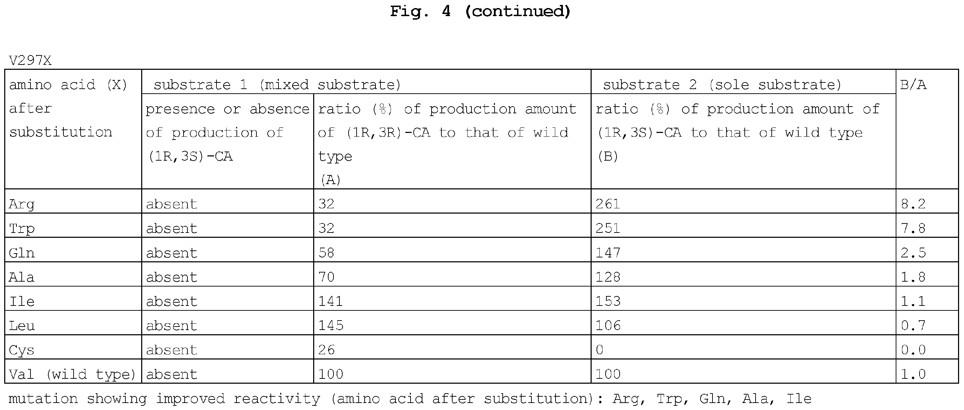

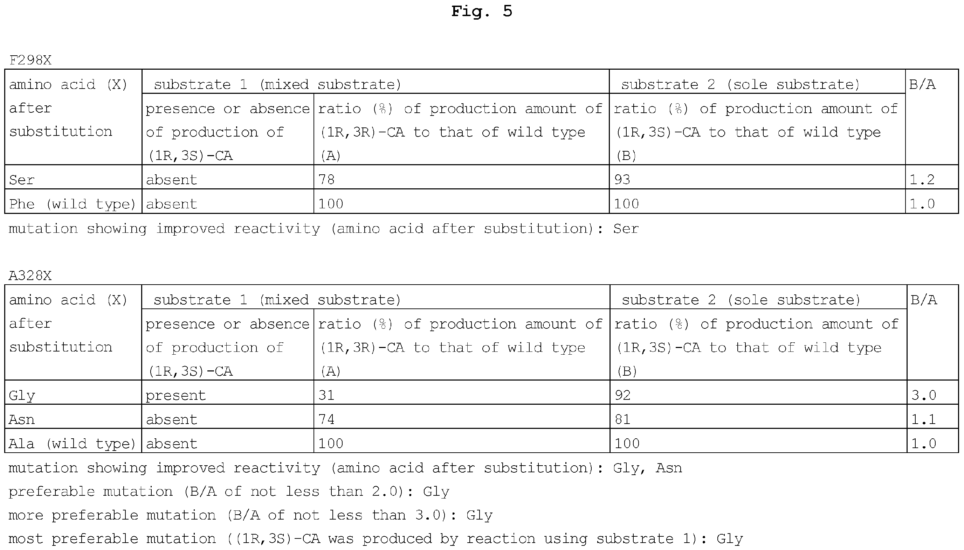

The evaluation results are shown in Figs. 1 to 5. The following mutations were identified to have increased the reactivity with (1R,3S)-ethyl chrysanthemate.
F58E, F58I, F58C, F58A, F58M, F58Y
D227I, D227S, D227A, D227W, D227H, D227G, D227F, D227V
I228Y, I228S, I228T, I228W
L229Y, L229F, L229V
L231M, L231A, L231G
S244F
A245N, A245G
V297R, V297W, V297Q, V297A, V297I
F298S
A328N, A328G

The following mutations showed values of not less than 2.0, and are more preferable for improving the reactivity with (1R,3S)-ethyl chrysanthemate.
F58E, F58I, F58C, F58A, F58M
D227I, D227S, D227A, D227W, D227H, D227G
I228Y, I228S
L229Y, L229F
L231M, L231A
V297R, V297W, V297Q
A328G

The value of the following mutations was not less than 3.0 and still more preferable. A328G showed production of (1R,3S)-chrysanthemic acid also in a reaction using substrate 1 (mixed substrate) and was a particularly effective mutation.
F58E, F58I, F58C, F58A, F58M
D227I, D227S, D227A, D227W, D227H
I228Y, I228S
L229Y
L231M
V297R, V297W
A328G

### 2-2. Evaluation of combinatorial variant

Substrate 1 (1 µL) was dispensed to a reaction container, a measurement sample (enzyme extract prepared in the above-mentioned 1.(3)) (99 µL) was added and reacted (40°C, 24 hr). After the reaction, 6N HCl (10 µL) and hexane (150 µL) were added. After thorough stirring, the mixture was centrifuged and the organic layer (100 µL) was collected. The extract was dried to solidness, re-dissolved in an analysis solvent, and analyzed by supercritical fluid chromatography (SFC). The analysis conditions were the same as those in the evaluation of CASTing Library.

Changes in the selectivity for (1R,3S)-ethyl chrysanthemate were evaluated based on the reactivity/selectivity of a mutated enzyme (A328G variant) having mutation (A328G) that improved selectivity for (1R,3S)-ethyl chrysanthemate as the standard. To be specific, a value of "a ratio (%) of production amount of (1R,3S)-chrysanthemic acid to that of A328G variant in a reaction using a mixed substrate"/"a ratio (%) of production amount of (1R,3R)-chrysanthemic acid to that of A328G variant in a reaction using a mixed substrate" was calculated from the SFC area. When the value was one or more, the mutation was judged to be effective in improving the selectivity for (1R,3S)-ethyl chrysanthemate.

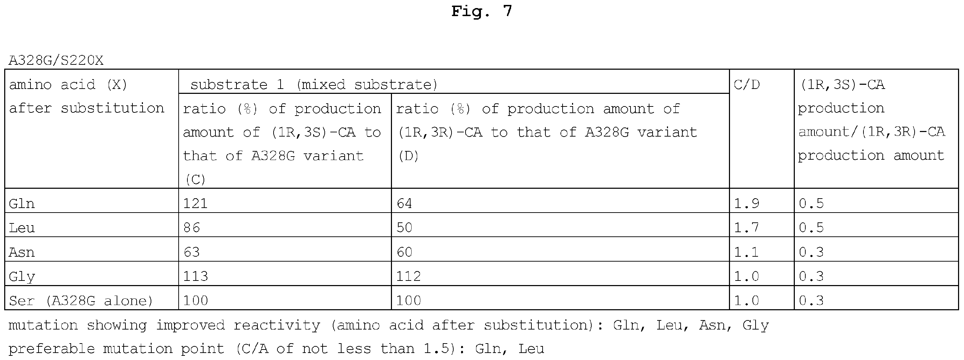

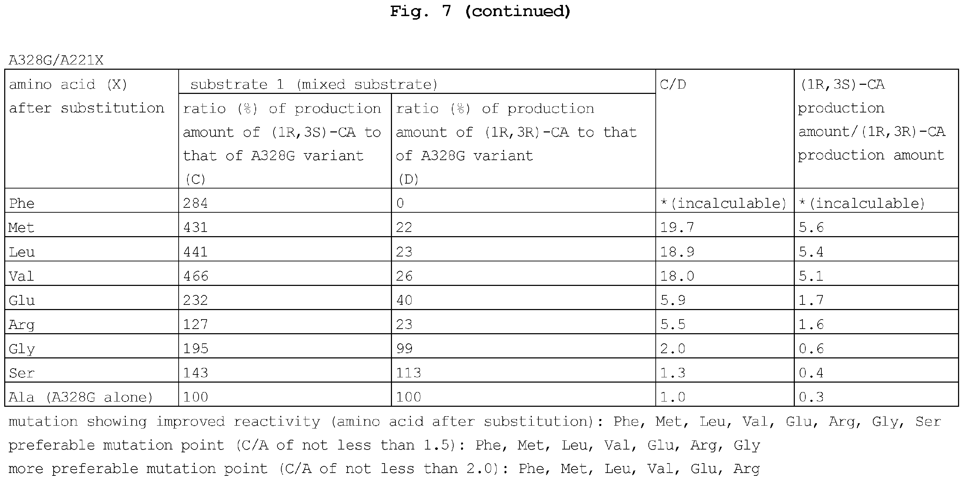

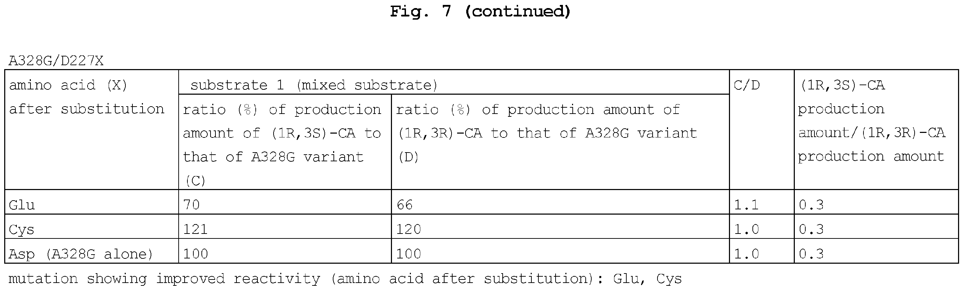

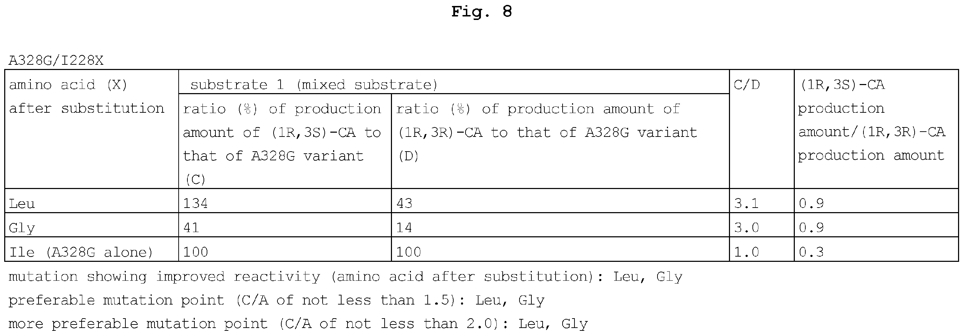

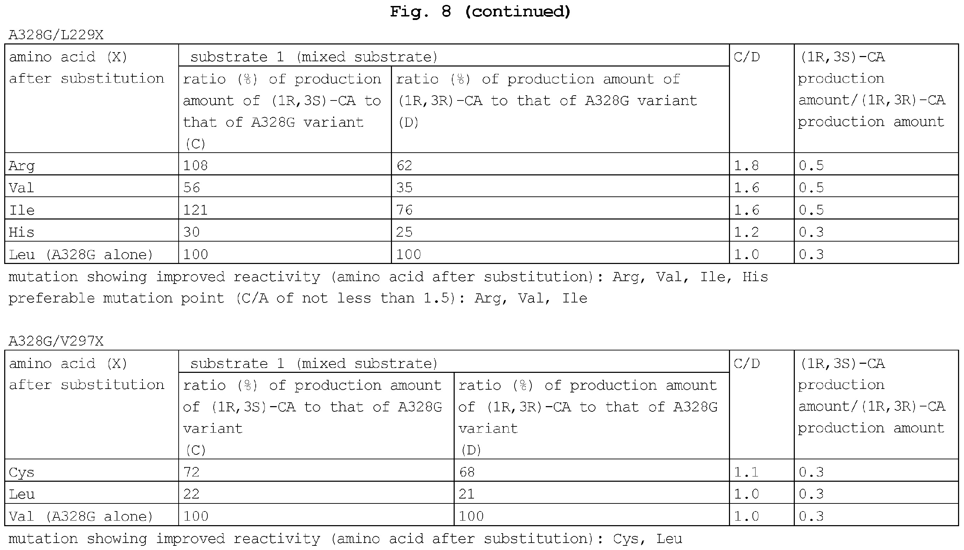

The evaluation results are shown in Figs. 6 to 8. The following combinations of mutations were identified to be effective in improving the selectivity for (1R,3S)-ethyl chrysanthemate. Surprisingly, as regards A328G/F217E, A328G/F217V, A328G/F217Y, A328G/S220Q, A328G/S220L, A328G/S220N and A328G/S220G, the reactivity was not improved when mutation point F217 or mutation point S220 was used solely, whereas the selectivity for (1R,3S)-ethyl chrysanthemate was improved when each mutation point was used in combination with A328G mutation.
A328G/F58L, A328G/F58E
A328G/L151Q, A328G/L151R, A328G/L151H, A328G/L151W, A328G/L151A,
A328G/L151V, A328G/L151M, A328G/L151N, A328G/L151C, A328G/L151T, A328G/L151I
A328G/F217W, A328G/F217G, A328G/F217A, A328G/F217D, A328G/F217T,
A328G/F217R, A328G/F217E, A328G/F217V, A328G/F217Y
A328G/S220Q, A328G/S220L, A328G/S220N, A328G/S220G
A328G/A221F, A328G/A221M, A328G/A221L, A328G/A221V, A328G/A221E,
A328G/A221R, A328G/A221G, A328G/A221S
A328G/D227E, A328G/D227C
A328G/I228L, A328G/I228G
A328G/L229R, A328G/L229V, A328G/L229I, A328G/L229H
A328G/V297C, A328G/V297L

The following combinations of mutations showed values of not less than 1.5, and are more preferable for improving the selectivity for (1R,3S)-ethyl chrysanthemate.
A328G/F58L
A328G/L151Q, A328G/L151R
A328G/F217W, A328G/F217G, A328G/F217A, A328G/F217D, A328G/F217T, A328G/F217R, A328G/F217E
A328G/S220Q, A328G/S220L
A328G/A221F, A328G/A221M, A328G/A221L, A328G/A221V, A328G/A221E, A328G/A221R, A328G/A221G
A328G/I228L, A328G/I228G
A328G/L229R, A328G/L229V, A328G/L229I

The following combinations of mutations showed values of not less than 2.0, and are further more preferable. Mutation A328G/A221F is to be particularly noted because it shows reactivity specific to (1R,3S)-ethyl chrysanthemate.
A328G/L151Q
A328G/F217W, A328G/F217G, A328G/F217A, A328G/F217D, A328G/F217T A328G/A221F, A328G/A221M, A328G/A221L, A328G/A221V, A328G/A221E, A328G/A221R
A328G/I228L, A328G/I228G

### 2-3. Evaluation of multiple variant

Substrate 1 (2 µL) was dispensed to a reaction container, a measurement sample (enzyme extract prepared in the above-mentioned 1.(3)) (98 µL) was added and reacted (40°C, 24 hr). After the reaction, 6N HCl (10 µL) and hexane (150 µL) were added. After thorough stirring, the mixture was centrifuged and the organic layer (100 µL) was collected. The extract was dried to solidness, re-dissolved in an analysis solvent, and analyzed by supercritical fluid chromatography (SFC). The analysis conditions were the same as those in the evaluation of CASTing Library.

Changes in the selectivity for (1R,3S)-ethyl chrysanthemate were evaluated based on the reactivity/selectivity of a mutated enzyme (A328G variant) having mutation (A328G) that improved selectivity for (1R,3S)-ethyl chrysanthemate as the standard. To be specific, a value of "a ratio (%) of production amount of (1R,3S)-chrysanthemic acid to that of A328G variant in a reaction using a mixed substrate"/"a ratio (%) of production amount of (1R,3R)-chrysanthemic acid to that of A328G variant in a reaction using a mixed substrate" was calculated from the SFC area. When the value was one or more, the mutation was judged to be effective in improving the selectivity for (1R,3S)-ethyl chrysanthemate, and a higher ratio in the production amount of (1R,3S)-ethyl chrysanthemate to that of A328G variant was judged to show a mutation more useful in improving the producibility of (1R,3S)-ethyl chrysanthemate.

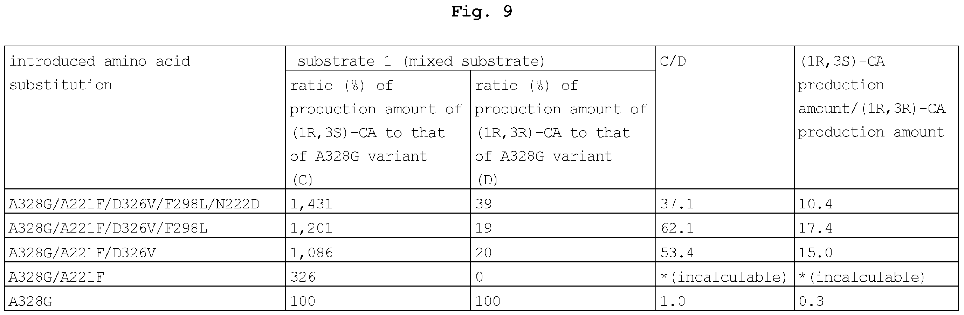

The evaluation results are shown in Fig. 9. The following combinations of mutations were identified to afford very high selectivity for and reactivity with (1R,3S)-ethyl chrysanthemate.
A328G/A221F/D326V
A328G/A221F/D326V/F298L
A328G/A221F/D326V/F298L/N222D

### 3. Summary

A plurality of mutations effective in improving the reactivity/selectivity for (1R,3S)-ethyl chrysanthemate have been successfully identified. The A328G/A221F variant shows (1R,3S)-ethyl chrysanthemate-specific reactivity and is extremely characteristic. The A328G/A221F/D326V/F298L/N222D variant shows markedly improved reactivity in addition to the selectivity for (1R,3S)-ethyl chrysanthemate, and is extremely useful. The amino acid sequences of the variants containing particularly effective mutations including these variants and genes encoding same (one embodiment for each) are shown below.
A328G variant: SEQ ID NO: 2 (amino acid sequence), SEQ ID NO: 13 (gene sequence)
A328G/F217W variant: SEQ ID NO: 3 (amino acid sequence), SEQ ID NO: 14 (gene sequence)
A328G/A221F variant: SEQ ID NO: 4 (amino acid sequence), SEQ ID NO: 15 (gene sequence)
A328G/A221M variant: SEQ ID NO: 5 (amino acid sequence), SEQ ID NO: 16 (gene sequence)
A328G/A221L variant: SEQ ID NO: 6 (amino acid sequence), SEQ ID NO: 17 (gene sequence)
A328G/A221V variant: SEQ ID NO: 7 (amino acid sequence), SEQ ID NO: 18 (gene sequence)
A328G/I228L variant: SEQ ID NO: 8 (amino acid sequence), SEQ ID NO: 19 (gene sequence)
A328G/A221F/D326V variant: SEQ ID NO: 9 (amino acid sequence), SEQ ID NO: 20 (gene sequence)
A328G/A221F/D326V/F298L variant: SEQ ID NO: 10 (amino acid sequence), SEQ ID NO: 21 (gene sequence)
A328G/A221F/D326V/F298L/N222D variant: SEQ ID NO: 11 (amino acid sequence), SEQ ID NO: 22 (gene sequence)

### [Industrial Applicability]

The modified esterase of the present invention has high reactivity with (1R,3S)-ethyl chrysanthemate and is useful for the production of (1R,3S)-chrysanthemic acid. Using the modified esterase of the present invention, (1R,3S)-chrysanthemic acid can be produced efficiently by an enzyme method, and the problems in the conventional production method (chemical hydrolysis) can be solved.

The present invention is not limited in any way to the description of the embodiments and examples of the above-described invention. The present invention also includes various modifications as long as they can be easily conceived by a person skilled in the art without departing from the description of the scope of the claims. The contents of the papers, published unexamined patent applications, patent publications, and the like explicitly indicated in the present specification are cited by reference in their entirety.

### [Sequence Listing Free Text]

SEQ ID NO: 2: explanation of artificial sequence: A328G variant
SEQ ID NO: 3: explanation of artificial sequence: A328G/F217W variant
SEQ ID NO: 4: explanation of artificial sequence: A328G/A221F variant
SEQ ID NO: 5: explanation of artificial sequence: A328G/A221M variant
SEQ ID NO: 6: explanation of artificial sequence: A328G/A221L variant
SEQ ID NO: 7: explanation of artificial sequence: A328G/A221V variant
SEQ ID NO: 8: explanation of artificial sequence: A328G/I228L variant
SEQ ID NO: 9: explanation of artificial sequence: A328G/A221F/D326V variant
SEQ ID NO: 10: explanation of artificial sequence: A328G/A221F/D326V/F298L variant
SEQ ID NO: 11: explanation of artificial sequence: A328G/A221F/D326V/F298L/N222D variant
SEQ ID NO: 13: explanation of artificial sequence: A328G variant
SEQ ID NO: 14: explanation of artificial sequence: A328G/F217W variant
SEQ ID NO: 15: explanation of artificial sequence: A328G/A221F variant
SEQ ID NO: 16: explanation of artificial sequence: A328G/A221M variant
SEQ ID NO: 17: explanation of artificial sequence: A328G/A221L variant
SEQ ID NO: 18: explanation of artificial sequence: A328G/A221V variant
SEQ ID NO: 19: explanation of artificial sequence: A328G/I228L variant
SEQ ID NO: 20: explanation of artificial sequence: A328G/A221F/D326V variant
SEQ ID NO: 21: explanation of artificial sequence: A328G/A221F/D326V/F298L variant
SEQ ID NO: 22: explanation of artificial sequence: A328G/A221F/D326V/F298L/N222D variant

## Claims

1. A modified esterase comprising an amino acid sequence resulting from an amino acid substitution of any one of the following (1) to (23) in the amino acid sequence of SEQ ID NO: 1, or an amino acid sequence having not less than 90% identity with the amino acid sequence (wherein the amino acid sequences are different in a portion other than the position of the amino acid substitution), the modified esterase having improved reactivity with (1R,3S)-ethyl chrysanthemate as compared to an esterase consisting of the amino acid sequence of SEQ ID NO: 1:
(1) mutation point: F58, amino acid after substitution: E, I, C, A, M or Y;
(2) mutation point: D227, amino acid after substitution: I, S, A, W, H, G, F or V;
(3) mutation point: 1228, amino acid after substitution: Y, S, T or W;
(4) mutation point: L229, amino acid after substitution: Y, F or V;
(5) mutation point: L231, amino acid after substitution: M, A or G;
(6) mutation point: S244, amino acid after substitution: F;
(7) mutation point: A245, amino acid after substitution: N or G;
(8) mutation point: V297, amino acid after substitution: R, W, Q, A or I;
(9) mutation point: F298, amino acid after substitution: S;
(10) mutation point: A328, amino acid after substitution: N;
(11) mutation point: A328, amino acid after substitution: G;
(12) mutation points: A328 and F58, amino acid at mutation point A328 after substitution: G, amino acid at mutation point F58 after substitution: L or E;
(13) mutation points: A328 and L151, amino acid at mutation point A328 after substitution: G, amino acid at mutation point L151 after substitution: Q, R, H, W, A, V, M, N, C, T or I;
(14) mutation points: A328 and F217, amino acid at mutation point A328 after substitution: G, amino acid at mutation point F217 after substitution: W, G, A, D, T, R, E, V or Y;
(15) mutation points: A328 and S220, amino acid at mutation point A328 after substitution: G, amino acid at mutation point S220 after substitution: Q, L, N or G;
(16) mutation points: A328 and A221, amino acid at mutation point A328 after substitution: G, amino acid at mutation point A221 after substitution: F, M, L, V, E, R, G or S;
(17) mutation points: A328 and D227, amino acid at mutation point A328 after substitution: G, amino acid at mutation point D227 after substitution: E or C;
(18) mutation points: A328 and 1228, amino acid at mutation point A328 after substitution: G, amino acid at mutation point 1228 after substitution: L or G;
(19) mutation points: A328 and L229, amino acid at mutation point A328 after substitution: G, amino acid at mutation point L229 after substitution: R, V, I or H;
(20) mutation points: A328 and V297, amino acid at mutation point A328 after substitution: G, amino acid at mutation point V297 after substitution: C or L;
(21) mutation points: A328, A221, and D326, amino acid at mutation point A328 after substitution: G, amino acid at mutation point A221 after substitution: F, amino acid at mutation point D326 after substitution: V;
(22) mutation points: A328, A221, D326, and F298, amino acid at mutation point A328 after substitution: G, amino acid at mutation point A221 after substitution: F, amino acid at mutation point D326 after substitution: V, an amino acid at mutation point F298 after substitution: L;
(23) mutation points A328, A221, D326, F298, and N222, amino acid at mutation point A328 after substitution: G, amino acid at mutation point A221 after substitution: F, amino acid at mutation point D326 after substitution: V, amino acid at mutation point F298 after substitution: L, amino acid at mutation point N222 after substitution: D.

2. The modified esterase according to claim 1, wherein the amino acid after substitution in (1) is E, I, C, A or M,
the amino acid after substitution in (2) is I, S, A, W, H or G,
the amino acid after substitution in (3) is Y or S,
the amino acid after substitution in (4) is Y or F,
the amino acid after substitution in (5) is M or A,
the amino acid after substitution in (8) is R, W or Q,
the amino acid at mutation point F58 after substitution in (12) is L,
the amino acid at mutation point L151 after substitution in (13) is Q or R,
the amino acid at mutation point F217 after substitution in (14) is W, G, A, D, T, R or E,
the amino acid at mutation point S220 after substitution in (15) is Q or L,
the amino acid at mutation point A221 after substitution in (16) is F, M, L, V, E, R or G,
the amino acid at mutation point L229 after substitution in (19) is R, V or I.

3. The modified esterase according to claim 1, wherein the amino acid after substitution in (4) is Y,
the amino acid after substitution in (5) is M,
the amino acid after substitution in (8) is R or W,
the amino acid at mutation point L151 after substitution in (13) is Q,
the amino acid at mutation point F217 after substitution in (14) is W, G, A, D or T,
the amino acid at mutation point A221 after substitution in (16) is F, M, L, V, E or R.

4. The modified esterase according to claim 1, wherein the amino acid substitution is any one of (11) to (23), and the modified esterase has improved selectivity for (1R,3S)-ethyl chrysanthemate.

5. The modified esterase according to claim 4, wherein the amino acid substitution is A328G, A328G/F217W, A328G/A221M, A328G/A221L, A328G/A221V, or A328G/I228L.

6. The modified esterase according to claim 4, wherein the amino acid substitution is A328G/A221F, and the modified esterase acts specifically for (1R,3S)-ethyl chrysanthemate.

7. The modified esterase according to claim 4, wherein the amino acid substitution is A328G/A221F/D326V.

8. The modified esterase according to claim 4, wherein the amino acid substitution is A328G/A221F/D326V/F298L.

9. The modified esterase according to claim 4, wherein the amino acid substitution is A328G/A221F/D326V/F298L/N222D.

10. The modified esterase according to any one of claims 1 to 9, wherein the identity is not less than 95%.

11. The modified esterase according to claim 1, consisting of the amino acid sequence of any of SEQ ID NOs: 2 to 11.

12. A gene encoding the modified esterase according to any one of claims 1 to 11.

13. The gene according to claim 12, comprising the base sequence of any of SEQ ID NOs: 13 to 22.

14. A recombinant DNA comprising the gene according to claim 12 or 13.

15. A microorganism harboring the recombinant DNA according to claim 14.

16. An enzyme preparation comprising the modified esterase according to any one of claims 1 to 11.

17. A method for preparing a modified esterase, comprising the following steps (I) to (III):
(I) a step of preparing a nucleic acid encoding an amino acid sequence of the modified esterase according to any one of claims 1 to 11;
(II) a step of expressing the nucleic acid, and
(III) a step of collecting an expression product.

18. The method according to claim 17, wherein the amino acid sequence is an amino acid sequence of any one of SEQ ID NOs: 2 to 11.

19. The method according to claim 17, wherein the nucleic acid comprises a base sequence of any of SEQ ID NOs: 13 to 22.

20. A method for producing (1R,3S)-chrysanthemic acid, comprising reacting the modified esterase according to any one of claims 1 to 11 with (1R,3S)-ethyl chrysanthemate.

21. The method according to claim 20, wherein the (1R,3S)-ethyl chrysanthemate coexists with (1R,3R)-ethyl chrysanthemate, (1S,3S)-ethyl chrysanthemate and (1S,3R)-ethyl chrysanthemate.
